# EUROPEAN PATENT APPLICATION

(11) **EP 1 555 327 A1**
(43) Date of publication of application: **20.07.2005**
(21) Application number: 03756657.7
(22) Date of filing: 16.10.2003
(51) Int. Cl.: C12Q 1/60, C12Q 1/26, C12Q 1/32, C12Q 1/44, G01N 33/92, C07J 1/00

(54) **METHOD AND REAGENT FOR MEASURING CHOLESTEROL IN HIGH-DENSITY LIPOPROTEINS**

(30) Priority: 16.10.2002 JP 2002301328
(71) Applicant: Kyowa Medex Co., Ltd., Chuo-ku, Tokyo 104-6004 (JP)
(72) Inventor: KATAYAMA, Yuki, Kyowa Medex Co. Ltd, Res. Lab., Sunto-gun, Shizuoka 411-0932 (JP); FUJINAKA, Mayumi, Kyowa Medex Co. Ltd, Res. Lab., Sunto-gun, Shizuoka 411-0932 (JP); MORIYAMA, Satoshi, Kyowa Yuka Co. Ltd, Res. Lab., Yokkaichi-shi, Mie 510-8502 (JP); MURATA, Shigeru, Kyowa Yuka Co. Ltd, Res. Lab., Yokkaichi-shi, Mie 510-8502 (JP)
(74) Representative: VOSSIUS & PARTNER
(86) International application number: PCT/JP2003/013259
(87) International publication number: WO 2004/035817

(57) **Abstract**

A method for quantitatively determining cholesterol in high-density lipoprotein, which comprises: treating a sample with cholesterol esterase and cholesterol oxidase or cholesterol esterase, an oxidized coenzyme and cholesterol dehydrogenase in an aqueous medium comprising a bile acid derivative; and measuring the formed hydrogen peroxide or a reduced coenzyme; and a reagent used therefor.

## Description

### Technical Field

The present invention relates to a method for quantitatively determining cholesterol in high-density lipoprotein (hereinafter, abbreviated as HDL) in a sample, a reagent therefor and a kit therefor, and also relates to novel bile acid derivatives and a process for producing the novel bile acid derivatives.

### Background Art

Depending upon their density, lipoproteins in living bodies are classified into high-density lipoprotein (HDL), low-density lipoprotein (hereinafter, abbreviated as LDL), very low-density lipoprotein (hereinafter, abbreviated as VLDL) and chylomicron (hereinafter, abbreviated as CM). Their functions in living body greatly differ mostly depending upon the difference in the kind of apoprotein and their lipid compositions vary as well. Among them, HDL relates to removal of cholesterol accumulated in cells by receiving cholesterol from various tissues including arterial wall and is a factor for the prevention of risk of various arteriosclerotic diseases such as coronary arteriosclerosis. HDL level in blood has been known to be useful for predicting the onset of arteriosclerotic diseases.

The conventional method for quantitatively determining cholesterol in HDL (hereinafter, abbreviated as HDL cholesterol) comprises two steps; a step of fractionation by an ultracentrifugal method, an immunochemical method, an electrophoretic method, a precipitation method, and the like; and a step of quantitative determination of cholesterol. However, the step of fractionation is complicated and takes a long time to operate, and moreover, there is a problem in terms of safety. Therefore, the measuring method accompanied by such step of fractionation is not suitable for practical use because it is too inefficient.

In recent years, various measuring methods have been developed for solving the aforementioned problems. For example, with regard to a measuring method where lipoproteins other than HDL are aggregated, there have been known a measuring method using a reagent which aggregates lipoproteins other than HDL such as dextran sulfate, a divalent metal salt and a chemically modified enzyme (Japanese Published Unexamined Patent Application No. 131197/1996), a measuring method using a reagent which forms a complex with lipoproteins other than HDL such as polyanion and a surfactant which does not dissolve lipoproteins such as a polyoxyethylene-polyoxypropylene copolymer (Japanese Published Unexamined Patent Application No. 201393/1996), a measuring method using a polyanion such as dextran sulfate, a divalent metal salt, a specific nonionic surfactant and albumin which is supplemented to the albumin contained in the sample (Japanese Published Unexamined Patent Application No. 285298/1997), and the like. There has been also known a measuring method for HDL cholesterol in serum or plasma comprising treating serum or plasma with a solution comprising a lipoprotein fractionating agent (a combination of polyanion such as dextran sulfate with divalent cation such as magnesium ion), without separating the resulting mixture solution into solid and liquid, treating the solution it with cholesterol esterase and cholesterol oxidase in the presence of anionic surfactant (alkyl sulfonate, bile acid or derivatives thereof), and measuring the formed hydrogen peroxide (Japanese Published Unexamined Patent Application No. 116996/1996).

In those methods for quantitatively determining HDL cholesterol where lipoproteins other than HDL are aggregated, they have a good correlation to the conventional standard method. However, there are problems such as inaccuracy due to turbidity by aggregates formed in the reaction and an excessive load to autoanalyzer due to deposition of metal hydroxide produced in washing of reaction cells by the reaction of metal salt in the reaction solution with an alkali used for washing of reaction cells.

With regard to a measuring method where lipoproteins other than HDL are not aggregated, there have been known methods such as a method where cholesterol in lipoproteins other than HDL is selectively converted to hydrogen peroxide using acylpolyoxyethylene sorbitan ester, the resulting hydrogen peroxide is eliminated and HDL cholesterol is enzymatically measured by adding polyoxyethylene alkyl ether (Japanese Published Unexamined Patent Application No. 299/1997), a method of HDL cholesterol comprising contacting a biological specimen with cholesterol esterase derived from pancreas, cholesterol oxidase and bile acid such as cholic acid in the presence of albumin, and measuring the compound which is consumed or produced by the enzymatic reaction of HDL cholesterol (International Publication WO 97/40376), and the like.

However, in those methods for quantitatively determining HDL cholesterol where lipoproteins other than HDL are not aggregated, there may be a problem of inaccuracy of measured value caused by an incomplete elimination of cholesterol in lipoproteins other than HDL or by a non-specific reaction with cholesterol in lipoproteins other than HDL.

With regard to a method for quantitatively determining HDL cholesterol using bile acid, there have been known, for example, a method comprising mixing serum or plasma in a buffer comprising cholesterol esterase and cholesterol oxidase, and a salt of bile acid, a bile acid derivative or dioctyl sulfosuccinate, with the enzymes to react cholesterol in VLDL and LDL prior to HDL cholesterol, measuring the formed hydrogen peroxide, adding a nonionic surfactant having polyoxyethylene oxide group to the reaction solution to react HDL cholesterol with the enzymes and measuring HDL cholesterol fractionately (Japanese Published Unexamined Patent Application No. 69999/1987); a method for quantitatively determining HDL cholesterol comprising reacting serum, in a buffer comprising pancreas-derived cholesterol esterase, cholesterol oxidase, a bile acid-type surfactant and a nonionic surfactant, with the enzymes at specific pH and specific temperature (Japanese Published Unexamined Patent Application No. 126498/1988); a method for quantitatively determining HDL cholesterol and LDL cholesterol comprising contacting a biological specimen with pancreas-derived cholesterol esterase and cholesterol oxidase in the presence of albumin and bile acid or a salt thereof to react HDL cholesterol with the enzymes and contacting the treated biological specimen with cholesterol esterase derived from microorganism to react with LDL cholesterol with the enzymes (microorganism-derived cholesterol esterase and cholesterol oxidase) (Japanese Published Unexamined Patent Application No. 9300/1999); a method for quantitatively determining HDL cholesterol and/or LDL cholesterol comprising reacting a sample comprising lipoproteins with enzymes (cholesterol esterase and cholesterol oxidase) in the presence of a bile acid derivative and/or amphoteric surfactant to react HDL cholesterol selectively and adding a nonionic surfactant having a polyoxyethylene chain to the reaction where HDL cholesterol selectively subjected to a reaction (Japanese Published Unexamined Patent Application No. 325097/2000); etc. in addition to the methods mentioned in the above Japanese Published Unexamined Patent Application No. 116996/1996 and International Publication WO 97/40376. In those measuring methods however, there are some cases where long time is needed for the measurement and, further, they are not always the methods that are specific to HDL cholesterol.

Although esters of cholic acid having an oxyethylene group in the ester moiety have been known already (Japanese Published Unexamined Patent Application No. 221941/1993), no method for quantitatively determining HDL cholesterol using such esters has been known.

With regard to a method for the synthesis of ester compounds, generally, a synthetic method conducted by a condensation reaction of carboxylic acid with alcohol is easy and convenient and, particularly, an azeotropic esterifying method using an acid catalyst has been often utilized. However, in syntheses of the compounds having a bulky substituent and/or water-soluble group, the yield is sometimes low (C. K. Ingold: "Structure and Mechanism in Organic Chemistry" (England), second edition, Bell, 1969, Chapter 15, pages 1128-1178). For example, in the aforementioned Japanese Published Unexamined Patent Application No. 221941/1993, a synthetic method for a cholate by the reaction of cholic acid with di(ethylene glycol) monomethyl ether using p-toluenesulfonic acid as a catalyst is described. However, even after refluxing for as long as 72 hours, yield of the cholate is as very low as 23% and, moreover, operations for after-treatment are complicated and troublesome.

In carboxylic acid having hydroxyl group in a molecule, an intramolecular esterification also proceeds and, therefore, after protecting the hydroxyl group in the molecule, then esterification with alcohol is carried out as well. For example, a method for the synthesis of ursodecholate using ursodecholic acid and 2-hydroxyethyloxyglucoside has been known (Japanese Published Unexamined Patent Application No. 199598/1999). The method as such includes 1) a step of protecting hydroxyl groups in ursodecholic acid by TBDMS (tert-butyldimethylsilyl) group, 2) a step of esterification and 3) a step of deprotection (removal of the TBDMS group), therefore that is hardly said to be a simple and easy synthetic method.

### Disclosure of the Invention

An object of the present invention is to provide a simple and accurate method for quantitatively determining cholesterol in high-density lipoprotein in a sample and to provide a reagent and a kit used for such a method.

The present invention relates to the following [1] to [42].
[1] A method for quantitatively determining cholesterol in high-density lipoprotein, which comprises:
   reacting a sample with cholesterol esterase and cholesterol oxidase or cholesterol esterase, an oxidized coenzyme and cholesterol dehydrogenase in an aqueous medium comprising a bile acid derivative; and
   measuring the formed hydrogen peroxide or a reduced coenzyme.
[2] The method according to [1], wherein the aqueous medium further comprises albumin.
[3] The method according to [1] or [2], wherein the cholesterol esterase is chemically modified cholesterol esterase.
[4] The method according to [3], wherein the chemically modified cholesterol esterase is cholesterol esterase which is modified by a group selected from the group consisting of a group having poly(ethylene glycol) as a main component, a group having poly(propylene glycol) as a main component, a group having a copolymer of poly(propylene glycol) and poly(ethylene glycol), a group having water-soluble polysaccharide, a sulfopropyl group, a sulfobutyl group, a polyurethane group and a group having a chelating function.
[5] The method according to [3], wherein the chemically modified cholesterol esterase is cholesterol esterase which is modified by a group having poly(ethylene glycol) as a main component.
[6] The method according to any one of [1] to [5], wherein the bile acid derivative is a bile acid derivative having an anionic surface activity.
[7] The method according to [6], wherein the bile acid derivative having an anionic surface activity is selected from the group consisting of cholic acid or a salt thereof, taurocholic acid or a salt thereof, glycocholic acid or a salt thereof, lithocholic acid or a salt thereof, deoxycholic acid or a salt thereof, chenodeoxycholic acid or a salt thereof, ursodeoxycholic acid or a salt thereof, 7-oxolithocholic acid or a salt thereof, 12-oxolithocholic acid or a salt thereof, 12-oxochenodeoxycholic acid or a salt thereof, 7-oxodeoxycholic acid or a salt thereof, hyocholic acid or a salt thereof, hyodeoxycholic acid or a salt thereof and dehydrocholic acid or a salt thereof.
[8] The method according to any one of [1] to [5], wherein the bile acid derivative is a bile acid derivative having an amphoteric surface activity.
[9] The method according to [8], wherein the bile acid derivative having an amphoteric surface activity is a compound represented by the formula (I)

   R¹-CH₂-CH(R²)-CH₂-SO₃⁻ (I)

   [wherein R¹ is a 3-(3-cholamidopropyl)dimethylammonio group and R² is a hydrogen atom or a hydroxyl group].
[10] The method according to any one of [1] to [5], wherein the bile acid derivative is a bile acid derivative having a nonionic surface activity.
[11] The method according to [10], wherein the bile acid derivative having a nonionic surface activity is a compound represented by the formula (II) 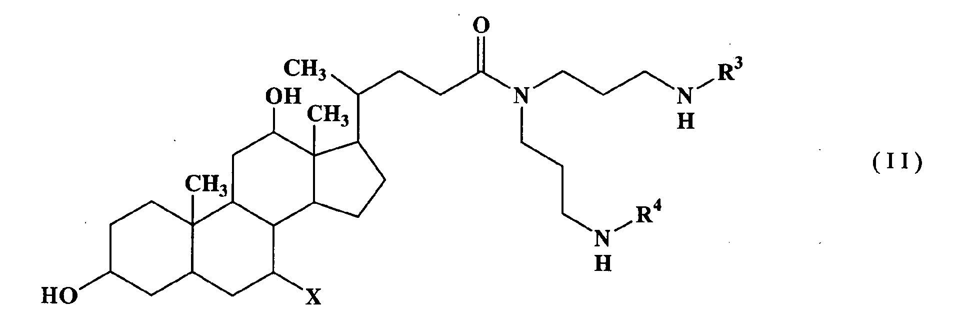 (wherein X is a hydrogen atom or a hydroxyl group; R³ and R⁴ may be the same or different and each represents a substituted or unsubstituted alkyl group or a substituted or unsubstituted alkanoyl group) or a compound represented by the formula (III) 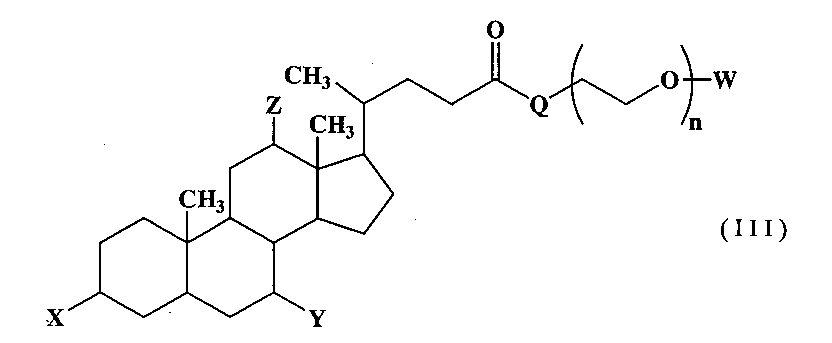 {wherein X, Y and Z maybe the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 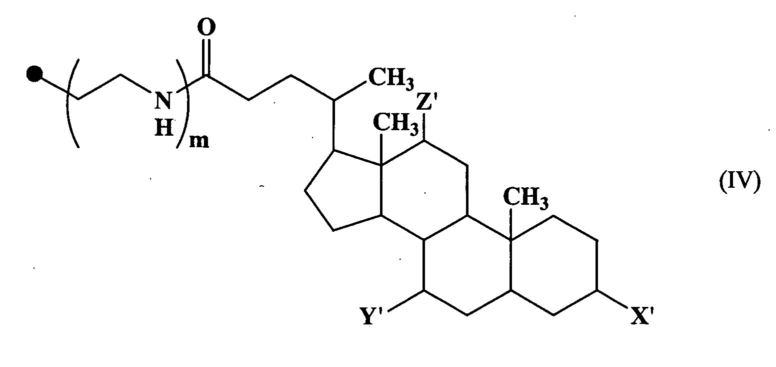 [wherein X', Y' and Z' may be the same or different and each represents a hydrogen atom, a hydroxyl group or an oxo (=O) group; and m is 0 or 1]; and n is an integer of 3 to 400}.
[12] A reagent for quantitatively determining cholesterol in high-density lipoprotein, which comprises cholesterol esterase, cholesterol oxidase, a bile acid derivative and a reagent for quantitatively determining hydrogen peroxide.
[13] A reagent for quantitatively determining cholesterol in high-density lipoprotein, which comprises cholesterol esterase, cholesterol dehydrogenase, a bile acid derivative and an oxidized coenzyme.
[14] The reagent according to [13], which further comprises a reagent for quantitatively determining a reduced coenzyme.
[15] The reagent according to any one of [12] to [14], which further comprises albumin.
[16] The reagent according to any one of [12] to [15], wherein the cholesterol esterase is chemically modified cholesterol esterase.
[17] The reagent according to [16], wherein the chemically modified cholesterol esterase is cholesterol esterase which is modified by a group selected from the group consisting of a group having poly (ethylene glycol) as a main component, a group having poly(propylene glycol) as a main component, a group having a copolymer of poly(propylene glycol) and poly(ethylene glycol), a group having a water-soluble polysaccharide, a sulfopropyl group, a sulfobutyl group, a polyurethane group and a group having a chelating function.
[18] The reagent according to [16], wherein the chemically modified cholesterol esterase is cholesterol esterase which is modified by a group having poly(ethylene glycol) as a main component.
[19] The reagent according to any one of [12] to [18], wherein the bile acid derivative is a bile acid derivative having an anionic surface activity.
[20] The reagent according to [19], wherein the bile acid derivative having an anionic surface activity is selected from the group consisting of cholic acid or a salt thereof, taurocholic acid or a salt thereof, glycocholic acid or a salt thereof, lithocholic acid or a salt thereof, deoxycholic acid or a salt thereof, chenodeoxycholic acid or a salt thereof, ursodeoxycholic acid or a salt thereof, 7-oxolithocholic acid or a salt thereof, 12-oxolithocholic acid or a salt thereof, 12-oxochenodeoxycholic acid or a salt thereof, 7-oxodeoxycholic acid or a salt thereof, hyocholic acid or a salt thereof, hyodeoxycholic acid or a salt thereof and dehydrocholic acid or a salt thereof.
[21] The reagent according to any one of [12] to [18], wherein the bile acid derivative is a bile acid derivative having an amphoteric surface activity.
[22] The reagent according to [21], wherein the bile acid derivative having an amphoteric surface activity is a compound represented by the formula (I)

   R¹-CH₂-CH(R²)-CH₂-SO₃⁻ (I)

   [wherein R¹ is a 3-(3-cholamidopropyl)dimethylammonio group and R² is a hydrogen atom or a hydroxyl group].
[23] The reagent according to any one of [12] to [18], wherein the bile acid derivative is a bile acid derivative having a nonionic surface activity.
[24] The reagent according to [23], wherein the bile acid derivative having a nonionic surface activity is a compound represented by the formula (II) 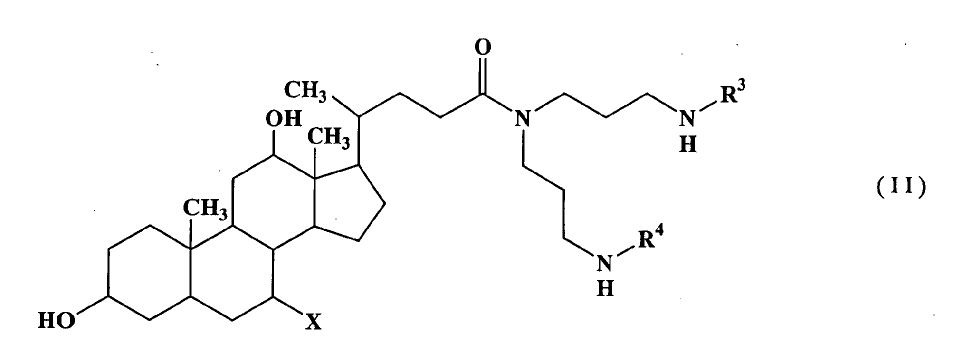 (wherein X is a hydrogen atom or a hydroxyl group; R³ and R⁴ may be the same or different and each represents a substituted or unsubstituted alkyl group or a substituted or unsubstituted alkanoyl group) or a compound represented by the formula (III) 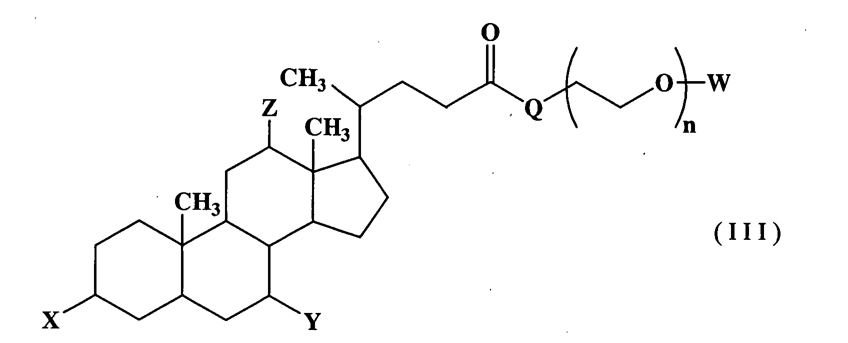 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 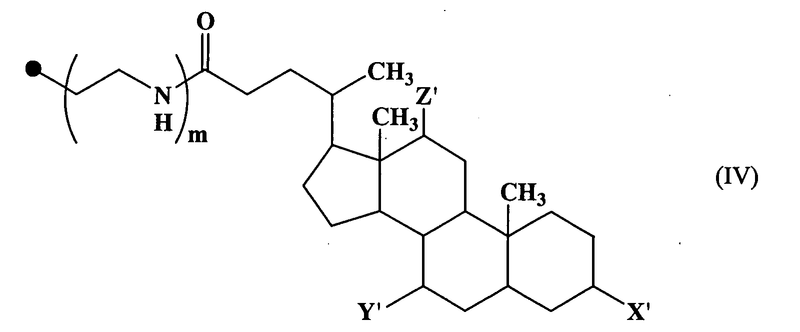 [wherein X', Y' and Z' may be the same or different and each represents a hydrogen atom, a hydroxyl group or an oxo (=O) group; and m is 0 or 1]; and n is an integer of 3 to 400}.
[25] A kit for quantitatively determining cholesterol in high-density lipoprotein, which comprises a first reagent comprising cholesterol esterase and a second reagent comprising cholesterol oxidase, wherein a bile acid derivative and a reagent for quantitatively determining hydrogen peroxide are comprised in either or both of the first reagent and/or the second reagent.
[26] A kit for quantitatively determining cholesterol in high-density lipoprotein, which comprises a first reagent comprising a bile acid derivative and a second reagent comprising cholesterol esterase and cholesterol oxidase, wherein a reagent for quantitatively determining hydrogen peroxide is contained in either or both the first reagent and/or the second reagent.
[27] A kit for quantitatively determining cholesterol in high-density lipoprotein, which comprises a first reagent comprising a reagent for quantitatively determining hydrogen peroxide a second reagent comprising cholesterol esterase and cholesterol oxidase, wherein a bile acid derivative is comprised in either or both of the first reagent and/or the second reagent.
[28] A kit for quantitatively determining cholesterol in high-density lipoprotein, which comprises a first reagent comprising cholesterol esterase and a second reagent comprising cholesterol dehydrogenase, wherein a bile acid derivative and an oxidized coenzyme are comprised in either or both of the first reagent and/or the second reagent.
[29] A kit for quantitatively determining cholesterol in high-density lipoprotein, which comprises a first reagent comprising a bile acid derivative and a second reagent comprising cholesterol esterase and cholesterol dehydrogenase, wherein an oxidized coenzyme is comprised in either or both of the first reagent and/or the second reagent.
[30] The kit according to [28] or [29], which further comprises a reagent for quantitatively determining a reduced coenzyme in either or both of the first reagent and/or the second reagent.
[31] The kit according to any one of [25] to [30], which further comprises albumin in either or both of the first reagent and/or the second reagent.
[32] The kit according to any one of [25] to [31], wherein the cholesterol esterase is chemically modified cholesterol esterase.
[33] The kit according to [32], wherein the chemically modified cholesterol esterase is cholesterol esterase which is modified by a group selected from the group consisting of a group having poly(ethylene glycol) as a main component, a group having poly(propylene glycol) as a main component, a group having a copolymer of poly(propylene glycol) and poly(ethylene glycol), a group having water-soluble polysaccharide, a sulfopropyl group, a sulfobutyl group, a polyurethane group and a group having a chelating function.
[34] The kit according to [32], wherein the chemically modified cholesterol esterase is cholesterol esterase which is modified by a group having poly(ethylene glycol) as a main component.
[35] The kit according to any one of [25] to [34], wherein the bile acid derivative is a bile acid derivative having an anionic surface activity.
[36] The kit according to [35], wherein the bile acid derivative having an anionic surface activity is selected from the group consisting of cholic acid or a salt thereof, taurocholic acid or a salt thereof, glycocholic acid or a salt thereof, lithocholic acid or a salt thereof, deoxycholic acid or a salt thereof, chenodeoxycholic acid or a salt thereof, ursodeoxycholic acid or a salt thereof, 7-oxolithocholic acid or a salt thereof, 12-oxolithocholic acid or a salt thereof, 12-oxochenodeoxycholic acid or a salt thereof, 7-oxodeoxycholic acid or a salt thereof, hyocholic acid or a salt thereof, hyodeoxycholic acid or a salt thereof and dehydrocholic acid or a salt thereof.
[37] The kit according to any one of [25] to [34], wherein the bile acid derivative is a bile acid derivative having an amphoteric surface activity.
[38] The kit according to [37], wherein the bile acid derivative having an amphoteric surface activity is a compound represented by the formula (I)

   R¹-CH₂-CH(R²)-CH₂-SO₃⁻ (I)

   [wherein R¹ is a 3-(3-cholamidopropyl)dimethylammonio group and R² is a hydrogen atom or a hydroxyl group].
[39] The kit according to any one of [25] to [34], wherein the bile acid derivative is a bile acid derivative having a nonionic surface activity.
[40] The kit according to [39], wherein the bile acid derivative having a nonionic surface activity is a compound represented by the formula (II) 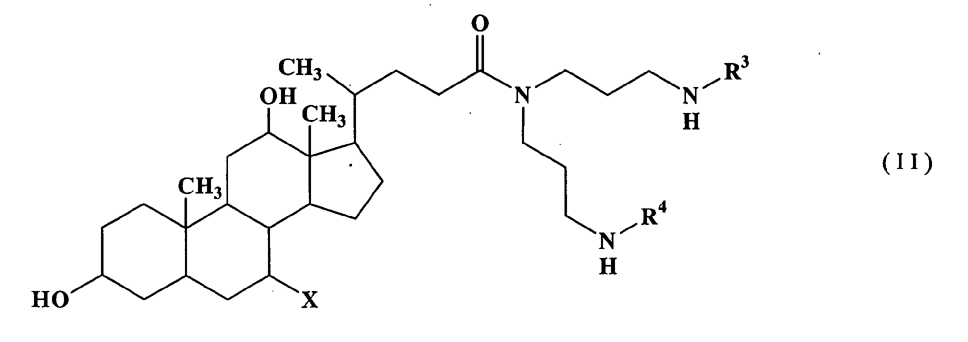 (wherein X is a hydrogen atom or a hydroxyl group; R³ and R⁴ may be the same or different and each represents a substituted or unsubstituted alkyl group or a substituted or unsubstituted alkanoyl group) or a compound represented by the formula (III) 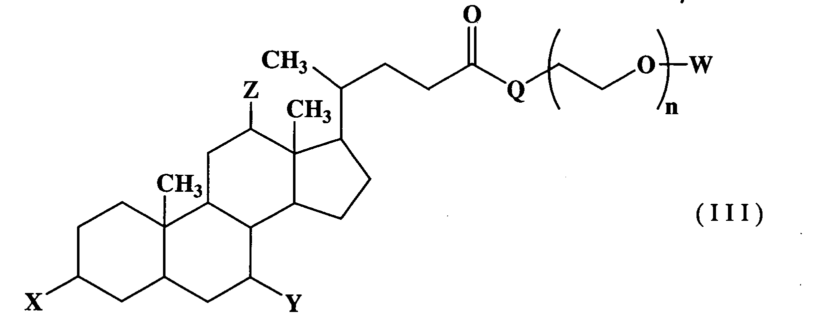 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 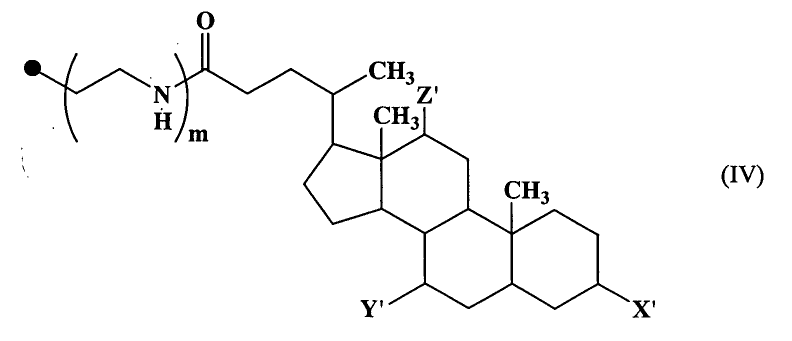 [wherein X', Y' and Z' may be the same or different and each represents a hydrogen atom, a hydroxyl group or an oxo (=O) group; and m is 0 or 1]; and n is an integer of 3 to 400}.
[41] A compound represented by the formula (III) 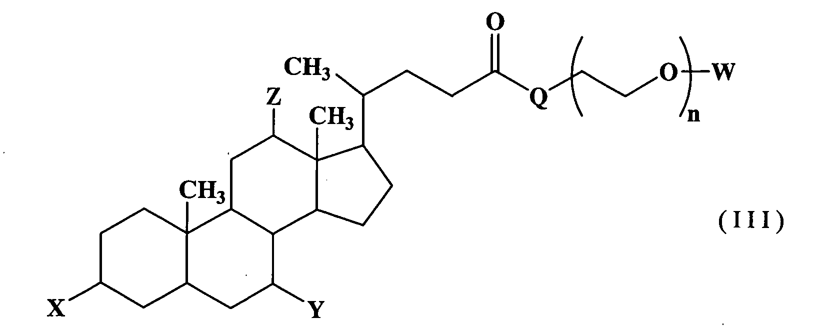 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 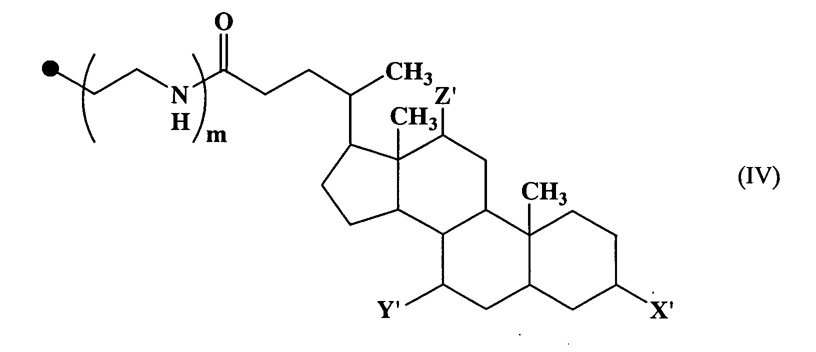 represents a hydrogen atom, a hydroxyl group or an oxo (=O) group; and m is 0 or 1]; and n is an integer of 3 to 400}.
[42] A process for producing a compound represented by the formula (III) 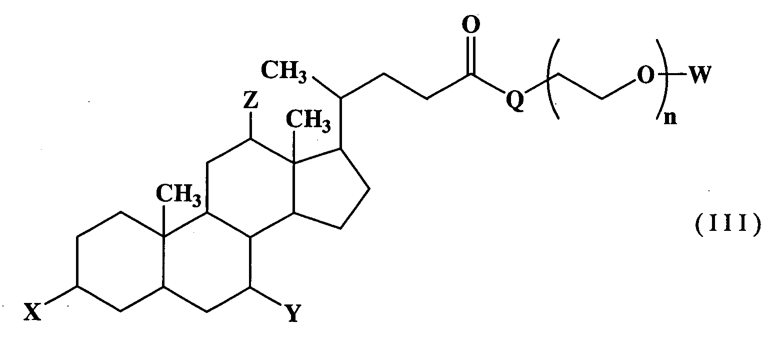
{wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 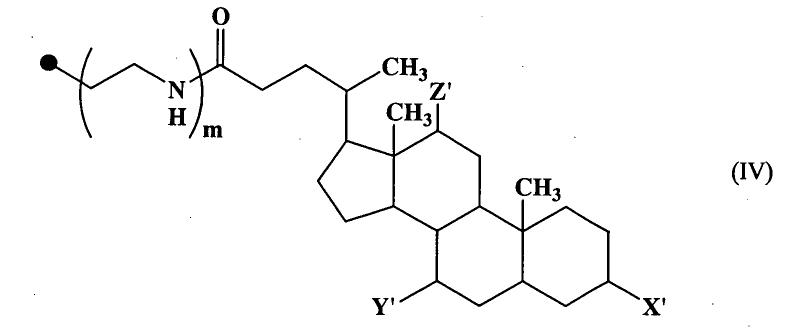 [wherein X', Y' and Z' may be the same or different and each represents a hydrogen atom, a hydroxyl group or an oxo (=O) group; and m is 0 or 1]; and n is an integer of 3 to 400}, which comprises:
reacting a compound represented by the formula (V) 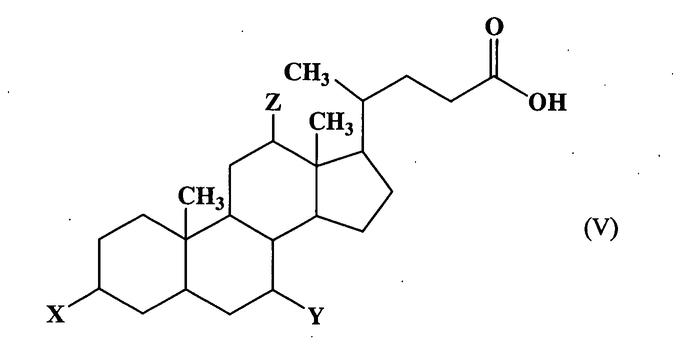
[wherein X, Y and Z maybe the same or different and each represents a hydrogen atom, a hydroxyl group or an oxo (=O) group] with a compound represented by the formula (VI) 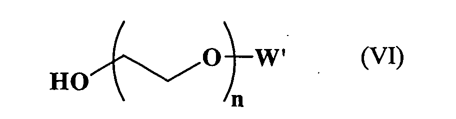 (wherein W' is a hydrogen atom, an alkyl group, an alkenyl group, an alkynyl group, a cycloalkyl group, a cycloalkenyl group, an alkanoyl group, an alkenoyl group, an alkynoyl group or a substituted or unsubstituted aryl group; and n is an integer of 3 to 400) or with a compound represented by the formula (VII) 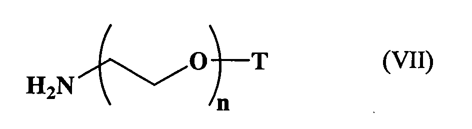 (wherein T is a substituted or unsubstituted aminoalkyl group; and n is an integer of 3 to 400) in the presence of a condensing agent.

The method for quantitatively determining HDL cholesterol according to the present invention is a method for quantitatively determining HDL cholesterol without eliminating cholesterol in lipoproteins other than HDL.

Examples of the sample used in the method according to the present invention are whole blood, plasma, serum, spinal fluid, saliva, amniotic fluid, urine, sweat, pancreatic fluid and the like and preferred ones are plasma and serum.

There is no particular limitation to the cholesterol esterase in the present invention so long as it is an enzyme having an ability to hydrolyze cholesterol ester, and it is possible to use, for example, cholesterol esterase and lipoprotein lipase derived from animals, plants and microorganisms as well as cholesterol esterase and lipoprotein lipase manufactured by means of genetic engineering.

With regard to the cholesterol esterase, both unmodified cholesterol esterase and chemically modified cholesterol esterase may be used. It is also possible to use cholesterol esterase which is available commercially.

Examples of the commercially available cholesterol esterase are cholesterol esterase "Amano" 2 (CHE2; manufactured by Amano Enzyme), cholesterol esterase "Amano" 3 (CHE3; manufactured by Amano Enzyme), lipoprotein lipase (LPL311; manufactured by Toyobo), lipoprotein lipase "Amano" 6 (LPL6; manufactured by Amano Enzyme), cholesterol esterase [COE313 (chemically modified cholesterol esterase); manufactured by Toyobo], and the like. It is also possible in the present invention to use two or more kinds of cholesterol esterases in combination.

Examples of the group which modifies the enzyme (chemically modifying group) in a chemical modification of cholesterol esterase include a group having poly(ethylene glycol) as a main component, a group having poly(propylene glycol)as a main component, a group having a copolymer of poly(propylene glycol) with poly(ethylene glycol), a group having water-soluble polysaccharide, a sulfopropyl group, a sulfobutyl group, a polyurethane group, a group having a chelating function and the like. Among them, preferred one is a group having poly (ethylene glycol) as a main component. Examples of the water-soluble polysaccharide are dextran, pullulan, soluble starch and the like.

Examples of the reagent which chemically modifies the cholesterol esterase (chemical modifier agent) are compounds which have both the above chemically modifying group and a functional group or structure reactive with an amino group, a carboxyl group, a sulfhydryl group, etc. in the enzyme. Examples of the functional group or the structure reactive with an amino group in the enzyme are a carboxyl group, an activated ester group (such as an N-hydroxysuccinimide group), acid anhydride, acid chloride, aldehyde, an epoxide group, 1,3-propane sultone, 1,4-butane sultone and the like. Examples of the functional group or the structure reactive with a carboxyl group in the enzyme are an amino group, and the like. Examples of the group or the structure reactive with sulfhydryl group in the enzyme are a maleimide group, disulfide, α-haloester (such as α-iodoester) and the like.

With regard to a chemical modifier, commercially available modifier may be used. Examples of the commercially available chemical modifier are Sunbright VFM-4101, Sunbright MEAC-50HS and Sunbright MEC-50HS having both an N-hydroxylsuccinimide group and a group having poly (ethylene glycol) as a main component (all manufactured by NOF), Sunbright AKM series (such as Sunbright AKM-1510), Sunbright ADM series, Sunbright ACM series having both an acid anhydride structure and a group having poly(alkylene glycol) as a main component (all manufactured by NOF), EPOX-3400 and M-EPOX-5000 having both an epoxide group and a group having poly(ethylene glycol) as a main component (all manufactured by Sheawater Polymers) and diethylenetriamine-N,N,N',N'',N''-pentaacetic acid anhydride (DTPA anhydride) having both an acid anhydride structure and a group having a chelating function (manufactured by Dojindo Laboratories).

Chemical modification of cholesterol esterase may, for example, be carried out by the following method although there is no limitation thereto. Firstly, cholesterol esterase is dissolved in a buffer of pH 8.0 or higher (such as HEPES buffer) and chemical modifier is added in an amount of 0.01 to 500-fold molar quantity of the enzyme at 0 to 55°C followed by stirring for 5 minutes to 5 hours. As chemically modified cholesterol esterase used in actual enzymatic reaction, not only the reaction solution as such but also a product where unreacted chemical modifier and the like are removed by, for example, ultrafiltration membrane if necessary may be used.

There is no particular limitation to the concentration of the cholesterol esterase used for the method of the present reaction so long as it enables the quantitative determination of HDL cholesterol of the present invention and the concentration in the reaction solution is preferably, 0.01 to 200 U/mL and, more preferably, 0.02 to 100 U/mL.

There is no particular limitation to the cholesterol oxidase in the present invention so long as it is an enzyme having an ability to produce hydrogen peroxide by oxidizing cholesterol. For example, in addition to cholesterol oxidase derived from animals, plants or microorganisms, it is possible to use cholesterol oxidase manufactured by means of genetic engineering. Commercially available ones such as cholesterol oxidase "Amano" 1 (CHOD1; manufactured by Amano Enzyme), cholesterol oxidase (CO-PE; manufactured by Kikkoman) and cholesterol oxidase (COO321; manufactured by Toyobo) may be used as well. In the present invention, two or more kinds of cholesterol oxidases may be used in combination.

The cholesterol oxidase may be either an unmodified enzyme or a chemically modified enzyme. Chemically modified cholesterol oxidase may, for example, be prepared by the above-mentioned chemically modifying method using the above-mentioned chemical modifier.

There is no particular limitation to the concentration of the cholesterol oxidase used for the method of the present reaction so long as it enables the quantitative determination of the HDL cholesterol of the present invention. It is preferred that a concentration in the reaction solution is 0.01 to 200 U/mL and, more preferably, 0.02 to 100 U/mL.

There is no particular limitation to the cholesterol dehydrogenase in the present invention so long as it is an enzyme having an ability to produce a reduced coenzyme by oxidation of cholesterol in the presence of an oxidized coenzyme. For example, in addition to cholesterol dehydrogenase derived from animals, plants and microorganisms, it is also possible to use cholesterol dehydrogenase manufactured by means of genetic engineering. Commercially available ones such as cholesterol dehydrogenase "Amano" 5 (CHDH5; manufactured by Amano) and the like may be used as well. In the present invention, two or more kinds of cholesterol dehydrogenases may be used in combination.

The cholesterol dehydrogenase may be either an unmodified enzyme or a chemically modified enzyme. Chemically modified cholesterol dehydrogenase may, for example, be prepared by the above-mentioned chemically modifying method using the above-mentioned chemical modifier.

There is no particular limitation to the concentration of the cholesterol dehydrogenase used for the method of the present reaction so long as it enables the quantitative determination of the HDL cholesterol of the present invention. It is preferred that a concentration in the reaction solution is 0.01 to 200 U/mL and, more preferably, 0.02 to 100 U/mL.

In a measuring method using the cholesterol dehydrogenase according to the present invention, an oxidized coenzyme is used. Examples of the oxidized coenzyme are NAD, NADP, thio-NAD, thio-NADP and the like.

There is no particular limitation to albumin used in the present invention so long as it is albumin that enables the quantitative determination of HDL cholesterol according to the present invention. Examples of the albumin are those derived from cattle, horse, sheep, human being and the like, and among them, bovine serum albumin (BSA) is preferred. It is also possible to use albumin manufactured by means of genetic engineering. In the present invention, two or more kinds of albumins having different origins may be used in combination. There is no particular limitation to the concentration of albumin in the quantitative determination of HDL cholesterol so long as it enables the quantitative determination of the HDL cholesterol of the present invention. It is preferred that a concentration in the reaction solution is 0.001 to 10% or, more preferably, 0.01 to 1%.

There is no particular limitation to the bile acid derivative used in the present invention so long as it enables the quantitative determination of the HDL cholesterol of the present invention. Examples of the bile acid derivative are a bile acid derivative having an anionic surface activity, a bile acid derivative having an amphoteric surface activity and a bile acid derivative having a nonionic surface activity and the like.

With regard to the bile acid derivative of the present invention, a bile acid derivative having a nonionic surface activity is particularly preferred in terms of accuracy or reproducibility of the quantitative determination of HDL cholesterol using enzymes such as cholesterol esterase, cholesterol oxidase and cholesterol esterase or in terms of stability on preservation of the enzyme for the quantitative determination of cholesterol.

Examples of the bile acid derivative having an anionic surface activity are cholic acid or a salt thereof, taurocholic acid or a salt thereof, glycocholic acid or a salt thereof, lithocholic acid or a salt thereof, deoxycholic acid or a salt thereof, chenodeoxycholic acid or a salt thereof, ursodeoxycholic acid or a salt thereof, 7-oxolithocholic acid or a salt thereof, 12-oxolithocholic acid or a salt thereof, 12-oxochenodeoxycholic acid or a salt thereof, 7-oxodeoxycholic acid or a salt thereof, hyocholic acid or a salt thereof, hyodeoxycholic acid or a salt thereof, dehydrocholic acid or a salt thereof and the like. Examples of the salt are an ammonium salt, a lithium salt, a sodium salt, a potassium salt, a magnesium salt, a calcium salt and the like. There is no particular limitation to the concentration of the bile acid derivative having an anionic surface activity so long as it enables the quantitative determination of the HDL cholesterol of the present invention. It is preferred that a concentration in the reaction solution is 0.001 to 30% or, more preferably, 0.01 to 3%.

With regard to a bile acid derivative having an amphoteric surface activity, a compound represented by the formula (I)

R¹-CH₂-CH(R²)-CH₂-SO₃⁻ (I)

[wherein R¹ is 3-(3-cholamidopropyl)dimethylammonio group and R² is hydrogen atom or hydroxyl group] [hereinafter, referred to as Compound (I)] is exemplified. Hereinafter, Compound (I) in which R² is hydrogen atom will be referred to as CHAPS and Compound (I) in which R² is hydroxyl group will be referred to as CHAPSO. There is no particular limitation to the concentration of the bile acid derivative having an amphoteric surface activity so long as it enables the quantitative determination of the HDL cholesterol of the present invention. It is preferred that a concentration in the reaction solution is 0.001 to 30% or, more preferably, 0.01 to 3%.

An example of the bile acid derivative having a nonionic surface activity is a compound represented by the formula (II) 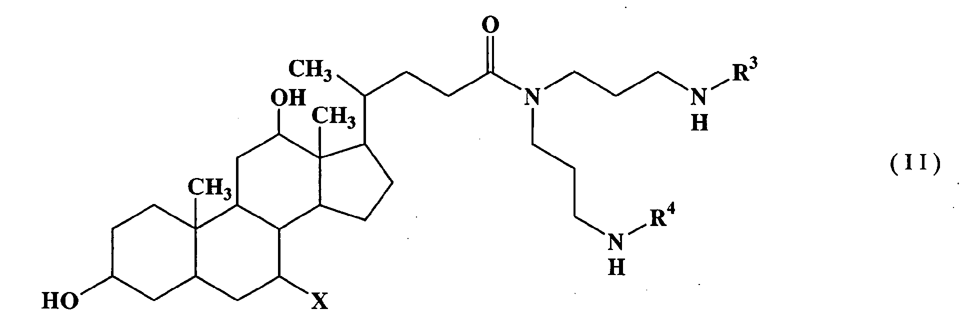 (wherein X is hydrogen atom or hydroxyl group; and R³ and R⁴ may be the same or different and each represents a substituted or unsubstituted alkyl or a substituted or unsubstituted alkanoyl) [hereinafter, referred to as Compound (II)] and the like. Examples of alkyl moieties in the alkyl group and the alkanoyl group include a straight or branched alkyl having 1 to 10 carbon atoms such as methyl, ethyl, propyl, isopropyl, butyl, isobutyl, sec-butyl, tert-butyl, pentyl, neopentyl, hexyl, heptyl, octyl, nonyl, decyl and the like. Examples of the substituent in the substituted alkyl group and the substituted alkanoyl group in Compound (II) are a hydroxyl group, a halogen atom and the like. The halogen atom means each of fluorine, chlorine, bromine and iodine atoms. In Compound (II), a compound where both R³ and R⁴ are

COCH(OH)CH(OH)CH(OH)CH(OH)CH₂OH

(hereinafter, referred to as Substituent A) is preferred. Hereinafter, a compound in which X, R³ and R⁴ are a hydrogen atom, Substituent A and Substituent A, respectively, will be referred to as deoxy-BIGCHAP while a compound in which X, R³ and R⁴ are a hydroxyl group, Substituent A and Substituent A, respectively, will be referred to as BIGCHAP.

Another example of the bile acid derivative having a nonionic surface activity is a compound [hereinafter, referred to as Compound (III)] represented by the formula (III) 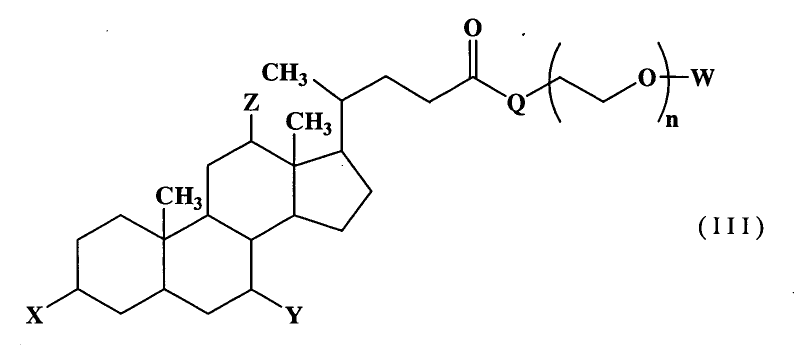 (wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group [hereinafter, referred to as Substituent (IV)] represented by the formula (IV) 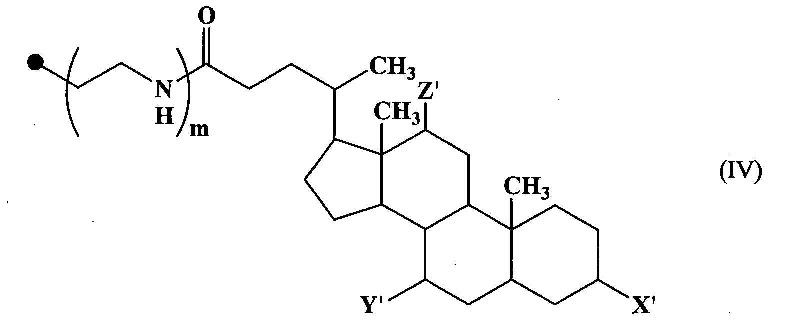 [wherein X', Y' and Z' may be the same or different and each represents a hydrogen atom, a hydroxyl group or an oxo (=O) group; and m is 0 or 1]; and n is an integer of 3 to 400}.

Compound (III) may be manufactured (synthesized) by, for example, reacting a compound [herein after, referred to as Compound (V)] represented by the formula (V) 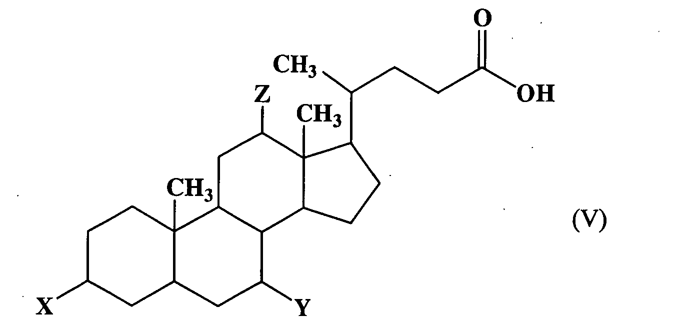 [wherein X, Y and Z may be the same or different and each represents a hydrogen atom, a hydroxyl group or an oxo (=O) group] is reacted in a solvent, in the presence of a condensing agent, with a compound [herein after referred to as Compound (IV)] represented by the formula (VI) 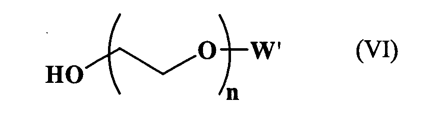 (wherein W' is a hydrogen atom, an alkyl group, an alkenyl group, an alkynyl group, a cycloalkyl group, a cycloalkenyl group, an alkanoyl group, an alkenoyl group, an alkynoyl group or a substituted or unsubstituted aryl group; and n is an integer of 3 to 400) or with a compound [hereinafter, referred to as Compound (VII)] represented by the formula (VII) 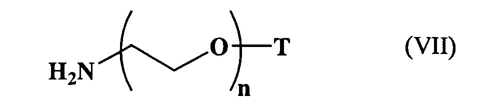 (wherein T is a substituted or unsubstituted aminoalkyl group; and n is an integer of 3 to 400). In the reaction, a base may be presented if necessary.

X, Y and Z each represents a hydrogen atom, a hydroxyl group or an oxo group and it is preferred that at least one of X, Y and Z is a hydroxyl group. X', Y' and Z' each represents a hydrogen atom, a hydroxyl group or an oxo group and it is preferred that at least one of X' , Y' and Z' is a hydroxyl group.

As an alkyl group which is common to the alkyl and the alkanoyl groups in Compound (III) and Compound (VI), a straight or branched group having 1 to 18 carbon atoms such as a methyl group, an ethyl group, a propyl group, an isopropyl group, a butyl group, an isobutyl group, a sec-butyl group, a tert-butyl group, a pentyl group, an isopentyl group, a neopentyl group, a hexyl group, an isohexyl group, a heptyl group, an isoheptyl group, an octyl group, an isooctyl group, a nonyl group, an isononyl group, a decyl group, an isodecyl group, an undecyl group, an isoundecyl group, a dodecyl group, an isododecyl group, a tridecyl group, an isotridecyl group, a tetradecyl group, an isotetradecyl group, a pentadecyl group, an isopentadecyl group, ahexadecacylgroup, an isohexadecacyl group , aheptadecylgroup, an isoheptadecyl group, an octadecacyl group, an isooctadecacyl group and the like are included. Among them, a methyl group, an ethyl group, a propyl group, a nonyl group and a dodecyl group are preferred.

As an alkenyl group which is common to the alkenyl and the alkenoyl groups in Compound (III) and Compound (VI), a straight or branched group having 2 to 8 carbon atoms such as a vinyl group, a propenyl group, an isopropenyl group, an allyl group, a butenyl group, a pentenyl group, a hexenyl group, a heptenyl group, an octenyl group and the like are included.

As an alkynyl which is common to the alkynyl and the alkynoyl groups in Compound (III) and Compound (VI), a straight or branched group having 2 to 8 carbon atoms such as an ethynyl group, a propynyl group, a propargyl group, a butynyl group, a pentynyl group, a hexynyl group, a heptynyl group, an octynyl group and the like are included.

As a cycloalkyl moieties in Compound (III) and Compound (VI), the group having 3 to 8 carbon atoms such as a cyclopropyl group, a cyclobutyl group, a cyclopentyl group, a cyclohexyl group, a cycloheptyl group, a cyclooctyl group and the like are included.

As a cycloalkenyl moieties in Compound (III) and Compound (VI), the group having 4 to 8 carbon atoms such as a cyclopropenyl group, a cyclobutenyl group, a cyclopentenyl group, a cyclohexenyl group, a cycloheptenyl group, a cyclooctenyl group and the like are included.

As an aryl group in Compound (III) and Compound (VI), the group having 6 to 15 carbon atoms such as a phenyl group, a naphthyl group, an anthryl group, a phenanthryl group, biphenyl group and the like are included. As a substituent in a substituted aryl group in Compound (III), an alkyl group, for example, is included. Examples of the alkyl group are the above-mentioned alkyl.

As the substituted aminoalkyl group in Compound (III) and Compound (VII), an N-monosubstituted aminoalkyl group, an N,N-disubstituted aminoalkyl group and the like are included. The two substituents on the nitrogen atoms in the N,N-disubstituted aminoalkyl group may be the same or different. An example of the substituent on a nitrogen atom in the N-monosubstituted aminoalkyl group and the N,N-disubstituted aminoalkyl group is the above-mentioned alkyl group. Specific examples of the N-monosubstituted aminoalkyl group are a 2-(N-methylamino)ethyl group, a2-(N-ethylamino)ethyl group, a2-(N-propylamino)ethyl group, a 2-(N-butylamino)ethyl group, a 3-(N-methylamino)propyl group, a 3-(N-ethylamino)propylgroup, a 3-(N-propylamino)propyl group, a 3-(N-butylamino)- propyl group, a 4-(N-methylamino)butyl group, a 4-(N-ethylamino) butyl group, a 4-(N-propylamino)butyl group, a 4-(N-butylamino)butyl group and the like. Specific examples of the N,N-disubstituted aminoalkyl group are 2-(N,N-dimethylamino) ethyl group, 3-(N,N-dimethylamino)propyl group, 4-(N,N-dimethylamino)butyl group and the like. Specific examples of the unsubstituted aminoalkyl group in Compound (III) and Compound (VII) are a 2-aminoethyl group, a 3-aminopropyl group, a 4-aminobutyl group and the like.

The factor n represents an average degree of polymerization of oxyethylene group in the ester moiety. There is no particular limitation to the average degree of polymerization so long as it is within such a range that enables the quantitative determination of HDL cholesterol of the present invention and it is preferably 3 to 400, more preferably 8 to 300, and, further preferably, 15 to 200.

Examples of Compound (V) are cholic acid, lithocholic acid, deoxycholic acid, chenodeoxycholic acid, ursodeoxycholic acid, 7-oxolithocholic acid, 12-oxolithocholic acid, 12-oxochenodeoxycholic acid, 7-oxodeoxycholic acid, hyocholic acid, hyodeoxycholic acid, dehydrocholic acid and the like.

In the synthesis of Compound (III) where W is a group selected from the group consisting of hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group and a substituted or unsubstituted aminoalkyl group [hereinafter, referred to as Compound (IIIa)], equivalent ratio of Compound (VI) or Compound (VII) to Compound (V) is preferably from 0.5 to 1.5 and, more preferably, from 1 to 1.2. In the synthesis of Compound (III) where W is a group represented by the formula (IV) [hereinafter, referred to as Compound (IIIb)], an equivalent ratio of Compound (V) to Compound (VI) or Compound (VII) is preferably from 1.3 to 4.1 and, more preferably, from 1.7 to 2.2.

With regard to the reaction temperature for the synthesis of Compound (III), it is preferably 10 to 40°C and, more preferably, 20 to 35°C. There is no particular limitation to the reaction solvent in the synthesis of Compound (III) so long as it is a solvent that does not disturb the condensation reaction of Compound (V) with Compound (VI), and its example is an organic solvent. Examples of the organic solvent are an aromatic hydrocarbon solvent such as toluene, xylene and the like, an aliphatic ester solvent such as ethyl acetate and the like and a halogenated hydrocarbon solvent such as dichloromethane, chloroform, dichloroethane and the like.

There is no particular limitation to a condensing agent used for the synthesis of Compound (III) so long as it progresses the condensation reaction of Compound (V) with Compound (VI) or Compound (VII). Examples of the condensing agent are carbodiimide, a pyridinium salt, a (benzo)thiazolium salt, a (benz)oxazolium salt, isocyanates, a carbonyl reagent and the like. Among them, the preferred one is carbodiimide. Examples of the carbodiimide are 1,3-dicyclohexylcarbodiimide, 1,3-di(tert-butyl)carbodiimide, 1,3-diisopropylcarbodiimide, water-soluble carbodiimide and the like and, in terms of easy removal by washing with water, the water-soluble carbodiimide is particularly preferred.

Examples of the water-soluble carbodiimide are 1-ethyl-3-(3-dimethylaminopropyl)carbodiimide and a salt thereof. Examples of the salt are a hydrochloride salt and a methyl iodide salt. Examples of the pyridinium salt are a 2-chloromethylpyridinium iodide salt and a 2-bromomethylpyridinium iodide salt. Examples of the (benz) oxazolium salt are a 2-chlorobenzoxazolium fluoroborate salt and the like. Examples of the (benzo)thiazolium salt are a 2-chlorobenzothiazolium fluoroborate salt and the like. Examples of the isocyanates are chlorosulfonyl isocyanate and the like. Examples of the carbonyl reagent are 1,1-carbonyldiimidazole and the like.

In the synthesis of Compound (IIIa), the equivalent number of the condensing agent in the condensation reaction is preferably from 1.0 to 5.0 equivalent (s) or, more preferably, from 1.1 to 2.5 equivalents to Compound (V). In the synthesis of Compound (IIIb), the equivalent number of a condensing agent is preferably from 1.0 to 5.0 equivalent (s) or, more preferably, from 1.1 to 2.5 equivalents to Compound (VI) or Compound (VII).

Examples of the base used, if necessary, in the synthesis of Compound (III) include, a pyridine analog and the like. Examples of the pyridine analog are pyridine, 2-picoline, 3-picoline, 4-picoline, 2-ethylpyridine, 3-ethylpyridine, 4-ethylpyridine, 2-propylpyridine, 3-propylpyridine, 4-propylpyridine, 2,6-lutidine, 2,3-lutidine, 2,4-lutidine, 2,5-lutidine, 3,4-lutidine, 3,5-lutidine, 2,4,6-collidine, 2,3,5-collidine, 2-dimethylaminopyridine, 4-dimethylaminopyridine, 4-pyrrolidinopyridine, 4-piperidinopyridine and the like, and particularly preferred ones are 4-dimethylaminopyridine, 4-pyrrolidinopyridine, 4-piperidinopyridine, and the like. It is preferred that the equivalent of the pyridine analog is from 0.01 to 2.5 equivalent(s) or, more preferably, from 0.7 to 1. 2 equivalent (s) to Compound (V).

With regard to an after-treatment for a condensation reaction of Compound (V) with Compound (VI) or Compound (VII), a series of operations comprising the addition of water or a mixed solution of water and organic solvent to the reaction solution, the extraction of Compound (III) [Compound (IIIa) or Compound (IIIb)] from the resulting reaction mixture with an organic solvent, removal of the water contained in the extracted solution by a drying agent, removal of the drying agent by filtration and evaporating the solvent in the resulting filtrate under ordinary or reduced pressure may be exemplified. It is also possible that an extracted solution comprising Compound (III) is washed with an acid so that basic substances (condensing agent, substances derived from condensing agent and base) comprised in the extracted solution are removed from the extracted solution. It is preferred that the extracted solution washed with the acid is further washed with water or brine. Although the organic solvent that is added to the reaction solution may be the same or different from the organic solvent used for the reaction, it is preferred to be the same. The organic solvent added to the reaction solution and the organic solvent used for the extraction may be same or different. With regard to the organic solvent and the organic solvent used for the extraction, the above-mentioned organic solvent may be listed and, among them, dichloromethane, ethyl acetate and the like are particularly preferred.

Although the reaction mixture itself comprising Compound (III) which is obtained by an after-treatment of a condensation reaction of Compound (V) with Compound (VI) or Compound (VII), may be used for quantitatively determining HDL cholesterol, a product purified appropriately by separating and purifying operations may also be used. Examples of the separating and purifying method are silica gel column chromatography, HPLC, distillation, fractional distillation, recrystallization and the like.

Although there is no particular limitation to the concentration of the bile acid derivative having a nonionic surface activity, it is preferred that a concentration in the reaction solution which enables the quantitative determination of HDL cholesterol of the present invention is 0.001 to 30% and, more preferably, 0.01 to 3%.

Examples of an aqueous medium used in the method for the quantitative determination of HDL cholesterol according to the present invention are deionized water, distilled water, a buffer solution and the like. Among them, a buffer solution is preferred. Examples of a buffer used for the buffer solution are a tris(hydroxymethyl)aminomethane buffer, a phosphate buffer, a borate buffer, a Good's buffer and the like. Examples of a Good's buffer are 2-Morpholinoethanesulfonic acid (MES), bis-(2-hydroxyethyl)iminotris(hydroxymethyl)methane (Bis-Tris), N-(2-acetamido)iminodiacetic acid (ADA), piperazine-N,N'-bis(2-ethanesulfonic acid) (PIPES), N-(2-acetamido)-2-aminoethanesulfonic acid (ACES), 3-morpholino-2-hydroxypropanesulfonic acid (MOPSO), N,N-bis(2-hydroxyethyl)-2-aminoethanesulfonic acid (BES), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), 3-[N,N-bis(2-hydroxyethyl)amino]-2-hydroxypropanesulfonic acid (DIPSO), N-[tris(hydroxymethyl)methyl]-2-hydroxy-3-aminopropanesulfonic acid (TAPSO), piperazine-N,N'-bis(2-hydroxypropanesulfonic acid) (POPSO), 3-[4-(2-hydroxyethyl)-1-piperazinyl]-2-hydroxypropanesulfonic acid (HEPPSO), 3-[4-(2-hydroxyethyl)-1-piperazinyl]propanesulfonic acid[(H)EPPS], N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO), N-cyclohexyl-3-aminopropanesulfonic acid (CAPS), and the like. There is no particular limitation to the concentration of the buffer so long as it is a concentration suitable for the measurement and it is preferably, 0.001 to 2.0 mol/L and, more preferably, 0.005 to 1.0 mol/L.

Hereinafter, a method for the quantitative determination of HDL cholesterol, a reagent for the quantitative determination therefor and a kit for the quantitative determination therefor according to the present invention will be described in detail.

### (Method for the quantitative determination of HDL cholesterol)

With regard to a method for quantitatively determining HDL cholesterol according to the present invention, methods of the following embodiments may be exemplified.

### Method for the measurement 1

HDL cholesterol in a sample can be quantitatively determined by
(1) reacting a sample with unmodified cholesterol esterase and cholesterol oxidase or with unmodified cholesterol esterase, an oxidized coenzyme and cholesterol dehydrogenase in an aqueous medium comprising a bile acid derivative,
(2) measuring the formed hydrogen peroxide or a reduced coenzyme, and
(3) calculating an HDL cholesterol concentration by correlating the value obtained in (2) and a previously-prepared calibration curve.

### Method for the measurement 2

HDL cholesterol in a sample can be quantitatively determined by
(1) reacting a sample with unmodified cholesterol esterase and cholesterol oxidase or with unmodified cholesterol esterase, an oxidized coenzyme and cholesterol dehydrogenase in an aqueous medium comprising a bile acid derivative and albumin,
(2) measuring the formed hydrogen peroxide or a reduced coenzyme, and
(3) calculating an HDL cholesterol concentration by correlating the value obtained in (2) and a previously-prepared calibration curve.

### Method for the measurement 3

HDL cholesterol in a sample can be quantitatively determined by
(1) reacting a sample with chemically modified cholesterol esterase and cholesterol oxidase or with chemically modified cholesterol esterase, an oxidized coenzyme and cholesterol dehydrogenase in an aqueous medium comprising a bile acid derivative,
(2) measuring the formed hydrogen peroxide or a reduced coenzyme, and
(3) calculating an HDL cholesterol concentration by correlating the value obtained in (2) and a previously-prepared calibration curve.

### Method for the measurement 4

HDL cholesterol in a sample can be quantitatively determined by
(1) reacting a sample with chemically modified cholesterol esterase and cholesterol oxidase or with chemically modified cholesterol esterase, an oxidized coenzyme and cholesterol dehydrogenase in an aqueous medium comprising a bile acid derivative and albumin,
(2) measuring the formed hydrogen peroxide or a reduced coenzyme, and
(3) calculating an HDL cholesterol concentration by correlating the value obtained in (2) and a previously-prepared calibration curve.

In the present methods for the measurement, the reaction of (1) is carried out at, for example, 10 to 50°C or, preferably, 20 to 40°C for 1 to 60 minutes or, preferably, 2 to 30 minutes.

Amount of the produced hydrogen peroxide can be measured by, for example, a reagent for quantitatively determining hydrogen peroxide. A reagent for quantitatively determining hydrogen peroxide is a reagent for converting the formed hydrogen peroxide to a detectable substance. With regard to a detectable substance, dye, luminescence, and the like may be exemplified, and dye is preferred. When the detectable substance is a dye, the reagent for quantitatively determining hydrogen peroxide comprises an oxidative-coloring type of chromogen and a peroxidative substance such as peroxidase and the like. Examples of the oxidative-coloring type of chromogen are oxidative-coloring type of chromogens mentioned later. When the detectable substance is luminescence, a reagent for quantitatively determining hydrogen peroxide comprises a chemiluminescent substance. Examples of the chemiluminescent substance are luminol, isoluminol, lucigenin, acridinium ester and the like.

When a reagent comprising an oxidative-coloring type of chromogen and peroxidative substance such as peroxidase is used as a reagent for quantitatively determining hydrogen peroxide, hydrogen peroxide can be quantitatively determined by reacting the hydrogen peroxide with an oxidative-coloring type chromogen in the presence of peroxidative substance to form a dye, and measuring the formed dye. When a reagent for quantitatively determining hydrogen peroxide comprising a chemiluminescent substance is used, hydrogen peroxide can be quantitatively determined by reacting the hydrogen peroxide with a chemiluminescent substance to form photon and, measuring the formed photon.

Examples of the oxidative-coloring type of chromogen are a leuco type of chromogen and an oxidative coupling-coloring type of chromogen and the like. A leuco type of chromogen is a substance that is solely converted to a dye in the presence of hydrogen peroxide and a peroxidative substance such as peroxidase and the like.

Specific examples are 10-N-carboxymethylcarbamoyl-3,7-bis(dimethylamino)-10H-Phenothiazine (CCAP), 10-N-methylcarbamoyl-3,7-bis(dimethylamino)-10H-phenothiazine (MCDP), N-(carboxymethylaminocarbonyl)-4,4'-bis(dimethylamino)-diphenylamine sodium salt (DA-64), 4,4'-bis(dimethylamino)-diphenylamine, bis[3-bis(4-chlorophenyl)methyl-4-dimethylaminophenyl]amine (BCMA) and the like.

An oxidative coupling-coloring type of chromogen is a substance which gives a dye by an oxidized coupling of two compounds in the presence of hydrogen peroxide and a peroxidative substance such asperoxidase and the like. Examples of a combination of two compounds are a combination of a coupler with an aniline compound and a combination of a coupler with a phenol compound. Examples of the coupler are 4-aminoantipyrine (4-AA), 3-methyl-2-benzothiazolinonehydrazide and the like. Examples of the aniline compound are N-3-sulfopropyl)aniline, N-ethyl-N-2-hydroxyl-3-sulfopropyl)-3-methylaniline (TOOS), N-ethyl-N-(2-hydroxy-3-sulfopropyl)-3,5-dimethylaniline (MAOS), N-ethyl-N-(2-hydroxy-3-sulfopropyl)-3,5-dimethoxyaniline (DAOS), N-ethyl-N-(3-sulfopropyl)-3-methylaniline (TOPS), N-(2-hydroxy-3-sulfopropyl)-3,5-dimethoxyaniline (HDAOS), N,N-dimethyl-3-methylaniline, N,N-di(3-sulfopropyl)-3,5-dimethoxyaniline, N-ethyl-N-(3-sulfopropyl)-3-methoxyaniline, N-ethyl-N-(3-sulfopropyl)aniline, N-ethyl-N-(3-sulfopropyl)-3,5-dimethoxyaniline, N-(3-sulfopropyl)-3,5-dimethoxyaniline, N-ethyl-N-(3-sulfopropyl)-3,5-dimethylaniline, N-ethyl-N-(2-hydroxy-3-sulfopropyl)-3-methoxyaniline, N-ethyl-N-(2-hydroxy-3-sulfopropyl)aniline, N-ethyl-N-(3-methylphenyl)-N'-succinylethylenediamine (EMSE), N-ethyl-N-(3-methylphenyl)-N'- acetylethylenediamine, N-ethyl-N-(2-hydroxy-3-sulfopropyl)-4-fluoro-3,5-dimethoxyaniline (F-DAOS) and the like. Examples of the phenol compound are phenol, 4-chlorophenol, 3-methylphenol, 3-hydroxy-2,4,6-triiodobenzoic acid (HTIB) and the like.

In the quantitative determination of hydrogen peroxide, there is no particular limitation to the concentration of the peroxidative substance so long as it is suitable for the measurement and, when peroxidase is used as the peroxidative substance, it is preferably, 1 to 100 kU/L. Although there is no particular limitation to the concentration of an oxidative-coloring type of chromogen so long as it is a concentration which is suitable for the measurement and it is preferably 0.01 to 10 g/L.

Examples of a method for quantitatively determining a reduced coenzyme are a method where absorbance of the formed reduced coenzyme is determined and a method using a reagent for quantitatively determining a reduced coenzyme is used. With regard to a wavelength used in a method for the measurement of absorbance of a reduced coenzyme, 300 to 500 nm is preferred, 330 to 400 nm is more preferred and around 340 nm is particularly preferred. The reagent for quantitatively determining a reduced coenzyme is a reagent which converts the formed reduced coenzyme into a detectable substance. Examples of the detectable substance are a dye and the like. An example of the reagent for quantitatively determining a reduced coenzyme when the detectable substance is a dye, is a reagent comprising diaphorase, an electron carrier and reductive-coloring type of chromogen. Examples of the electron carrier are 1-methoxy-5-methylphenazium methyl sulfate, and the like. When a reagent comprising diaphorase, an electron carrier and a reductive-coloring type of chromogen is used as a reagent for quantitatively determining a reduced coenzyme, the reduced coenzyme can be quantitatively determined by measuring a dye which is given by conversion of the reductive-coloring type of chromogen.

Examples of the reductive-coloring type of chromogen are 3-(4,5-dimethyl-2-thiazolyl)-2,5-diphenyl-2H-tetrazolium bromide (MTT), 2-(4-iodophenyl)-3-(4-nitrophenyl)-5-(2,4-disulfophenyl)-2H-tetrazolium monosodium salt (WST-1), 2-(4-iodophenyl)-3-(2,4-dinitrophenyl)-5-(2,4-disulfophenyl)-2H-tetrazolium monosodium salt (WST-3) and the like.

### (Reagent for quantitatively determining HDL cholesterol)

Examples of the reagent for quantitatively determining HDL cholesterol according to the present invention are the reagents of the following embodiments.

### Reagent 1

A reagent comprising unmodified cholesterol esterase, cholesterol oxidase, a bile acid derivative and a reagent for quantitatively determining hydrogen peroxide.

### Reagent 2

A reagent comprising unmodified cholesterol esterase, cholesterol oxidase, a bile acid derivative, albumin and a reagent for quantitatively determining hydrogen peroxide.

### Reagent 3

A reagent comprising unmodified cholesterol esterase, cholesterol oxidase, a bile acid derivative and an oxidized coenzyme.

### Reagent 4

A reagent comprising unmodified cholesterol esterase, cholesterol oxidase, a bile acid derivative, albumin and an oxidized coenzyme.

### Reagent 5

A reagent comprising unmodified cholesterol esterase, cholesterol dehydrogenase, a bile acid derivative, an oxidized coenzyme and a reagent for quantitatively determining a reduced coenzyme.

### Reagent 6

A reagent comprising unmodified cholesterol esterase, cholesterol dehydrogenase, a bile acid derivative, albumin, an oxidized coenzyme and a reagent for quantitatively determining a reduced coenzyme.

### Reagent 7

A reagent comprising chemically-modified cholesterol esterase, cholesterol oxidase, a bile acid derivative and a reagent for quantitatively determining hydrogen peroxide.

### Reagent 8

A reagent comprising chemically-modified cholesterol esterase, cholesterol oxidase, a bile acid derivative, albumin and a reagent for quantitatively determining hydrogen peroxide.

### Reagent 9

A reagent comprising chemically-modified cholesterol esterase, cholesterol dehydrogenase, a bile acid derivative and an oxidized coenzyme.

### Reagent 10

A reagent comprising chemically-modified cholesterol esterase, cholesterol dehydrogenase, a bile acid derivative, albumin and an oxidized coenzyme.

### Reagent 11

A reagent comprising chemically-modified cholesterol esterase, cholesterol dehydrogenase, a bile acid derivative, an oxidized coenzyme and a reagent for quantitatively determining a reduced coenzyme.

### Reagent 12

A reagent comprising chemically modified cholesterol esterase, cholesterol dehydrogenase, a bile acid derivative, albumin, an oxidized coenzyme and a reagent for quantitatively determining a reduced coenzyme.

In the reagent for quantitatively determining HDL cholesterol according to the present invention, cholesterol esterase, cholesterol oxidase, cholesterol dehydrogenase, a bile acid derivative, albumin, a reagent for quantitatively determining hydrogen peroxide, an oxidized coenzyme and a reagent for quantitatively determining a reduced coenzyme which are mentioned in the above-mentioned method for the quantitative determination of HDL cholesterol according to the present invention may be used.

### (Kit for quantitatively determining HDL cholesterol)

The reagent for quantitatively determining HDL cholesterol according to the present invention may be preserved, circulated and used in a form of a kit. There is no particular limitation to a form of a kit and, although any of a two-reagent system, a three-reagent system, etc. may be acceptable, a two-reagent system is preferred.

In a kit for quantitatively determining HDL cholesterol of a two-reagent system comprising the first reagent and the second reagent, cholesterol esterase and cholesterol oxidase or cholesterol dehydrogenase may be comprised in the first reagent and the second reagent separately or may be comprised together in the second reagent and, in case they are comprised in the first reagent and the second reagent separately, an embodiment where cholesterol esterase is comprised in the first reagent while cholesterol oxidase or cholesterol dehydrogenase is comprised in the second reagent is preferred. A bile acid derivative may be comprised in either or both of the first reagent and/or the second reagent. An oxidized coenzyme used in a measuring method using cholesterol dehydrogenase may be comprised in either or both of the first reagent and/or the second reagent. A Reagent for quantitatively determining hydrogen peroxide may be comprised in either or both of the first reagent and/or the second reagent and, when the reagent comprises a chromogen of an oxidative coupling type, an embodiment where each compound of the chromogen of an oxidative coupling type is comprised in different reagent is preferred. A reagent for quantitatively determining a reduced coenzyme may be comprised in either or both of the first reagent and/or the second reagent. Albumin may be comprised in either or both of the first reagent and/or the second reagent.

To be more specific, kits of the following embodiments will be exemplified.

### Kit 1

### First reagent

A reagent comprising unmodified cholesterol esterase, a bile acid derivative and a reagent for quantitatively determining hydrogen peroxide.

### Second reagent

A reagent comprising cholesterol oxidase and a reagent for quantitatively determining hydrogen peroxide.

### Kit 2

### First reagent

A reagent comprising unmodified cholesterol esterase, a bile acid derivative, albumin and a reagent for quantitatively determining hydrogen peroxide.

### Second reagent

A reagent comprising cholesterol oxidase and a reagent for quantitatively determining hydrogen peroxide.

### Kit 3

### First reagent

A reagent comprising chemically modified cholesterol esterase, a bile acid derivative and a reagent for quantitatively determining hydrogen peroxide.

### Second reagent

A reagent comprising cholesterol oxidase and a reagent for quantitatively determining hydrogen peroxide.

### Kit 4

### First reagent

A reagent comprising chemically modified cholesterol esterase, a bile acid derivative, albumin and a reagent for quantitatively determining hydrogen peroxide.

### Second reagent

A reagent comprising cholesterol oxidase and a reagent for quantitatively determining hydrogen peroxide.

### Kit 5

### First reagent

A reagent comprising a reagent for quantitatively determining hydrogen peroxide.

### Second reagent

A reagent comprising unmodified cholesterol esterase, cholesterol oxidase, a bile acid derivative and a reagent for quantitatively determining hydrogen peroxide.

### Kit 6

### First reagent

A reagent comprising albumin and a reagent for quantitatively determining hydrogen peroxide.

### Second reagent

A reagent comprising unmodified cholesterol esterase, cholesterol oxidase, a bile acid derivative and a reagent for quantitatively determining hydrogen peroxide.

### Kit 7

### First reagent

A reagent comprising a reagent for quantitatively determining hydrogen peroxide.

### Second reagent

A reagent comprising chemically modified cholesterol esterase, cholesterol oxidase, a bile acid derivative and a reagent for quantitatively determining hydrogen peroxide.

### Kit 8

### First reagent

A reagent comprising albumin and a reagent for quantitatively determining hydrogen peroxide.

### Second reagent

A reagent comprising chemically modified cholesterol esterase, cholesterol oxidase, a bile acid derivative and a reagent for quantitatively determining hydrogen peroxide.

### Kit 9

### First reagent

A reagent comprising unmodified cholesterol esterase and a bile acid derivative.

### Second reagent

A reagent comprising cholesterol dehydrogenase and an oxidized coenzyme.

### Kit 10

### First reagent

A reagent comprising unmodified cholesterol esterase, a bile acid derivative and albumin.

### Second reagent

A reagent comprising cholesterol dehydrogenase and an oxidized coenzyme.

### Kit 11

### First reagent

A reagent comprising unmodified cholesterol esterase and a bile acid derivative.

### Second reagent

A reagent comprising cholesterol dehydrogenase, an oxidized coenzyme and a reagent for quantitatively determining a reduced coenzyme.

### Kit 12

### First reagent

A reagent comprising unmodified cholesterol esterase, a bile acid derivative and albumin.

### Second reagent

A reagent comprising cholesterol dehydrogenase, an oxidized coenzyme and a reagent for quantitatively determining a reduced coenzyme.

### Kit 13

### First reagent

A reagent comprising chemically modified cholesterol esterase and a bile acid derivative.

### Second reagent

A reagent comprising cholesterol dehydrogenase and an oxidized coenzyme.

### Kit 14

### First reagent

A reagent comprising chemically modified cholesterol esterase, a bile acid derivative and albumin.

### Second reagent

A reagent comprising cholesterol dehydrogenase and an oxidized coenzyme.

### Kit 15

### First reagent

A reagent comprising chemically modified cholesterol esterase and a bile acid derivative.

### Second reagent

A reagent comprising cholesterol dehydrogenase, an oxidized coenzyme and a reagent for quantitatively determining a reduced coenzyme.

### Kit 16

### First reagent

A reagent comprising chemically modified cholesterol esterase, a bile acid derivative and albumin.

### Second reagent

A reagent comprising cholesterol dehydrogenase, an oxidized coenzyme and a reagent for quantitatively determining a reduced coenzyme.

### Kit 17

### First reagent

A reagent comprising a bile acid derivative.

### Second reagent

A reagent comprising unmodified cholesterol esterase, cholesterol dehydrogenase and an oxidized coenzyme.

### Kit 18

### First reagent

A reagent comprising a bile acid derivative and albumin

### Second reagent.

A reagent comprising unmodified cholesterol esterase, cholesterol dehydrogenase and an oxidized coenzyme.

### Kit 19

### First reagent

A reagent comprising a bile acid derivative.

### Second reagent

A reagent comprising unmodified cholesterol esterase, cholesterol dehydrogenase, an oxidized coenzyme and a reagent for quantitatively determining a reduced coenzyme.

### Kit 20

### First reagent

A reagent comprising a bile acid derivative and albumin.

### Second reagent

A reagent comprising unmodified cholesterol esterase, cholesterol dehydrogenase, an oxidized coenzyme and a reagent for quantitatively determining a reduced coenzyme.

### Kit 21

### First reagent

A reagent comprising a bile acid derivative.

### Second reagent

A reagent comprising chemically modified cholesterol esterase, cholesterol dehydrogenase and an oxidized coenzyme.

### Kit 22

### First reagent

A reagent comprising a bile acid derivative and albumin.

### Second reagent

A reagent comprising chemically modified cholesterol esterase, cholesterol dehydrogenase and an oxidized coenzyme.

### Kit 23

### First reagent

A reagent comprising a bile acid derivative.

### Second reagent

A reagent comprising chemically modified cholesterol esterase, cholesterol dehydrogenase, an oxidized coenzyme and a reagent for quantitatively determining a reduced coenzyme.

### Kit 24

### First reagent

A reagent comprising a bile acid derivative and albumin.

### Second reagent

A reagent comprising chemically modified cholesterol esterase, cholesterol dehydrogenase, an oxidized coenzyme and a reagent for quantitatively determining a reduced coenzyme.

In the kit for quantitatively determining HDL cholesterol according to the present invention, cholesterol esterase, cholesterol oxidase, cholesterol dehydrogenase, a bile acid derivative, albumin, a reagent for quantitatively determining hydrogen peroxide, an oxidized coenzyme and a reagent for quantitatively determining a reduced coenzyme which are mentioned in the above-mentioned method for the quantitative determination of HDL cholesterol according to the present invention may be used.

If necessary, the reagent for quantitatively determining HDL cholesterol and the kit for quantitative determination therefor according to the present invention may further comprise an aqueous medium, a stabilizing agent, an antiseptic agent, an interfering substance elimination agent, a reaction promoter and the like. Examples of the aqueous medium are the above-mentioned aqueous medium and the like. Examples of the stabilizing agent are ethylenediamine tetraacetic acid (EDTA), sucrose, calcium chloride and the like. Examples of the antiseptic agent are sodium azide, antibiotic substance and the like. Examples of the interfering substance elimination agent are ascorbate oxidase for elimination of interference by ascorbic acid and the like. Examples of the reaction promoter are enzymes such as colipase, phospholipase and the like and salts such as sodium sulfate, sodium chloride and the like. The reagent for quantitatively determining HDL cholesterol and the kit therefor according to the present invention may be in a form of the lyophilized state or in a state of being dissolved in an aqueous medium. When HDL cholesterol in a sample is measured using a reagent in a form of the lyophilized state, the reagent is dissolved in an aqueous medium prior to use, and the dissolved reagent is used for the measurement.

With regard to the amount of cholesterol esterase, cholesterol oxidase and cholesterol dehydrogenase in the reagent for quantitatively determining HDL cholesterol and in the kit for the quantitative determination of the same according to the present invention, the amount that gives the concentration of enzymes at 0.01 to 800 U/mL is preferred and more preferably at 0.02 to 400 U/mL when dissolved in an aqueous medium. With regard to the amount of a bile acid derivative in the reagent for quantitatively determining HDL cholesterol and in the kit for the quantitative determination of the same according to the present invention, the amount that gives the concentration of a bile acid derivative at 0.001 to 30% is preferred and more preferably at 0.01 to 10% when dissolved in an aqueous medium. With regard to the amount of albumin in the reagent for quantitatively determining HDL cholesterol and in the kit for the quantitative determination of the same according to the present invention, the amount that gives the concentration of albumin at 0.001 to 10% is preferred and more preferably at 0.01 to 5% is when dissolved in an aqueous medium.

The present invention will now be illustrated in more detail in the following Examples although they are never intended to limit the scope of the present invention. Incidentally, in the present Examples, the following reagents and enzymes were used.

HEPES (manufactured by BDH Laboratory), EMSE (manufactured by Daito Chemix Corporation), bovine serum albumin (BSA; manufactured by Oriental Yeast), 4-aminoantipyrine (manufactured by Saikyo Kasei), peroxidase (manufactured by Toyobo), LPL311 (cholesterol esterase; manufactured by Toyobo), LPL6 (cholesterol esterase; manufactured by Amano Enzyme), CHE2 (cholesterol esterase; manufactured by Amano Enzyme), COE313 (chemically modified cholesterol esterase; manufactured by Toyobo), COO321 (cholesterol oxidase; manufactured by Toyobo), sodium cholate (manufactured by ACROS), sodium taurocholate (manufactured by Tokyo Kasei), BIGCHAP (manufactured by Dojindo Laboratories), CHAPS (manufactured by Dojindo Laboratories) and sodium dextran sulfate (molecular weight: 500,000; manufactured by Pharmacia).

In identification of a bile acid derivative in the present Examples, the following analytical instruments were used for acquiring various data.

Nuclear magnetic resonance spectrum (¹H-NMR, ¹³C-NMR); JOEL GSX-400 (manufactured by Nippon Denshi)

In the measurement of ¹H-NMR spectrum, tetramethylsilane is used as an internal standard and chemical shift (δ) is shown where tetramethylsilane is a standard. In the measurement of ¹³C-NMR spectrum, chemical shift (δ) of each peak is shown when central peak of three peaks derived from CDCl₃ is adjusted to 77.0 ppm.
Infrared absorption spectrum (IR): Nicolet NEXUS 470 (manufactured by Nicolet)
Gel permeation chromatography (GPC):
   System: Tosoh HLC-8120 GPC (manufactured by Tosoh)
   GPC column: Two TSK gel Super H-RC (for low-molecular analysis) (manufactured by Tosoh) were connected in series.
Column retention temperature: 40°C
Developing solvent: tetrahydrofuran
Flow rate: 0.5 mL/minute
Detector: RI (refractive index)
Standard substance for the preparation of calibration curve: poly(ethylene glycol)

### Best Mode for Carrying Out the Invention

### Reference Example 1: Preparation of chemically modified LPL311

After LPL311 was added to a HEPES buffer (pH 8.5; 0.15 mol/L) to be 33 g/L and cooled to 5°C, Sanbright VFM-4101, Sanbright AKM-1510 or Sanbright MEAC-50HS (all manufactured by NFO) was added thereto to be 330 g/L and allowed to react for 3 hours. The resulting modified enzyme solution was not purified/separated but used as chemically modified LPL311 just as it was.

### Reference Example 2: Preparation of chemically modified CHE2

After CHE2 was added to a HEPES buffer (pH 8.5; 0.15 mol/L) to be 50 g/L and cooled to 5°C, Sunbright VFM-4101 (manufactured by NOF) was added thereto to be 200 g/L and allowed to react for 3 hours. The resulting modified enzyme solution was not purified/separated but used as chemically modified CHE2 just as it was.

### Example 1: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| Sodium cholate | 6.0 g/L |

### Example 2: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| Sodium cholate | 6.0 g/L |

### Example 3: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| Sodium taurocholate | 7.0 g/L |

### Example 4: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| Sodium taurocholate | 7.0 g/L |

### Example 5: Kit for quantitatively determining HDL cholesterol

A kit for quantitatively determining of HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| BIGCHAP | 4.5 g/L |

### Example 6: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| BIGCHAP | 4.5 g/L |

### Example 7: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| CHAPS | 6.0 g/L |

### Example 8: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |
| CHAPS | 6.0 g/L |

### Comparative Example 1: Kit for quantitatively determining of HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |

### Comparative Example 2: Kit for quantitatively determining HDL cholesterol

A kit for quantitatively determining of HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 3.0 kU/L |

### Example 9: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |
| Sodium cholate | 6.0 g/L |

### Example 10: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |
| Sodium cholate | 6.0 g/L |

### Example 11: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |
| Sodium taurocholate | 7.0 g/L |

### Example 12: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10-mmol/L |
| 4-Aminoantipyrine | 0.3 g/L |
| Peroxidase | 20 kU/L |
| LPL6 | 0.05 kU/L |
| COO321 | 3.0 kU/L |
| Sodium taurocholate | 7.0 g/L |

### Example 13: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |
| BIGCHAP | 4.5 g/L |

### Example 14: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |
| BIGCHAP | 4.5 g/L |

### Example 15: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |
| CHAPS | 6.0 g/L |

### Example 16: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |
| CHAPS | 6.0 g/L |

### Comparative Example 3: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |

### Comparative Example 4: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| LPL6 | 0.05 kU/L |
| COO321 | 3.0 kU/L |

### Example 17: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified CHE2(modified by VFM-4101) | 0.2 kU/L |
| COO321 | 3.0 kU/L |
| Sodium cholate | 6.0 g/L |

### Example 18: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by MEAC-50HS) | 0.2 kU/L |
| COO321 | 3.0 kU/L |
| Sodium cholate | 6.0 g/L |

### Example 19: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by AKM-1510) | 0.2 kU/L |
| COO321 | 3.0 kU/L |
| Sodium cholate | 6.0 g/L |

### Example 20: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| COE313 | 1.0 kU/L |
| COO321 | 3.0 kU/L |
| Sodium cholate | 6.0 g/L |

### Example 21: Quantitative determination of HDL cholesterol

HDL cholesterol in 30 samples of human serum was measured by a Hitachi 7170 autoanalyzer using the kit of Example 1.

### (1) Preparation of calibration curve

A calibration curve showing the relation between HDL cholesterol concentration and "absorbance" was prepared by Hitachi 7170 autoanalyzer using a physiological brine (HDL cholesterol concentration: 0.0 mg/dL) and serum (HDL cholesterol concentration: 60.0 mg/dL) as standard solutions and the kit of Example 1 as a kit.

"Absorbance" used here means a value obtained by subtracting E1 from E2 on the basis of the two absorbances (E1 and E2) measured by the following reaction.

A standard solution (3 µL) and the first reagent (0.24 mL) were added to a reaction cell and heated at 37°C for 5 minutes, absorbance (E1) of the reaction solution was measured at a main wavelength of 600 nm and a sub-wavelength of 700 nm, the second reagent (0.08 mL) was added to the reaction solution followed by heating at 37°C for 5 minutes and absorbance (E2) of the reaction solution was measured at a main wavelength of 600 nm and a sub-wavelength of 700 nm.

### (2) Calculation of "absorbance" in a human serum sample by the reaction of the sample with the kit of Example 1

The same method as in the calculation of "absorbance" in (1) was conducted except that human serum sample was used instead of the standard solution used in the preparation of a calibration curve in (1) whereupon "absorbance" in the sample was calculated.

### (3) Quantitative determination of HDL cholesterol concentration in human serum sample

HDL cholesterol concentration in each sample was determined by correlating the "absorbance" calculated in (2) and the calibration curve prepared in (1).

### Example 22: Quantitative determination of HDL cholesterol

The same operation as in the measuring method of Example 21 was conducted except that the kit of Example 2 was used instead of the kit of Example 1 whereupon HDL cholesterol in 30 human serum samples was measured using Hitachi 7170 autoanalyzer.

### Example 23: Quantitative determination of HDL cholesterol

The same operation as in the measuring method of Example 21 was conducted except that the kit of Example 3 was used instead of the kit of Example 1 whereupon HDL cholesterol in 30 human serum samples was measured using Hitachi 7170 autoanalyzer.

### Example 24: Quantitative determination of HDL cholesterol

The same operation as in the measuring method of Example 21 was conducted except that the kit of Example 4 was used instead of the kit of Example 1 whereupon HDL cholesterol in 30 human serum samples was measured using Hitachi 7170 autoanalyzer.

### Example 25: Quantitative determination of HDL cholesterol

The same operation as in the measuring method of Example 21 was conducted except that the kit of Example 5 was used instead of the kit of Example 1 whereupon HDL cholesterol in 30 human serum samples was measured using Hitachi 7170 autoanalyzer.

### Example 26: Quantitative determination of HDL cholesterol

The same operation as in the measuring method of Example 21 was conducted except that the kit of Example 6 was used instead of the kit of Example 1 whereupon HDL cholesterol in 30 human serum samples was measured using Hitachi 7170 autoanalyzer.

### Example 27: Quantitative determination of HDL cholesterol

The same operation as in the measuring method of Example 21 was conducted except that the kit of Example 7 was used instead of the kit of Example 1 whereupon HDL cholesterol in 30 human serum samples was measured using Hitachi 7170 autoanalyzer.

### Example 28: Quantitative determination of HDL cholesterol

The same operation as in the measuring method of Example 21 was conducted except that the kit of Example 8 was used instead of the kit of Example 1 whereupon HDL cholesterol in 30 human serum samples was measured using Hitachi 7170 autoanalyzer.

### Comparative Example 5: Quantitative determination of HDL cholesterol

The same operation as in the measuring method of Example 21 was conducted except that the kit of Comparative Example 1 was used instead of the kit of Example 1 whereupon HDL cholesterol in 30 human serum samples was measured using Hitachi 7170 autoanalyzer.

### Comparative Example 6: Quantitative determination of HDL cholesterol

The same operation as in the measuring method of Example 21 was conducted except that the kit of Comparative Example 2 was used instead of the kit of Example 1 whereupon HDL cholesterol in 30 human serum samples was measured using Hitachi 7170 autoanalyzer.

### Example 29: Quantitative determination of HDL cholesterol

The same operation as in the measuring method of Example 21 was conducted except that the kit of Example 9 was used instead of the kit of Example 1 whereupon HDL cholesterol in 30 human serum samples was measured using Hitachi 7170 auto analyzer.

### Example 30: Quantitative determination of HDL cholesterol

The same operation as in the measuring method of Example 21 was conducted except that the kit of Example 10 was used instead of the kit of Example 1 whereupon HDL cholesterol in 30 human serum samples was measured using Hitachi 7170 autoanalyzer.

### Example 31: Quantitative determination of HDL cholesterol

The same operation as in the measuring method of Example 21 was conducted except that the kit of Example 11 was used instead of the kit of Example 1 whereupon HDL cholesterol in 30 human serum samples was measured using Hitachi 7170 autoanalyzer.

### Example 32: Quantitative determination of HDL cholesterol

The same operation as in the measuring method of Example 21 was conducted except that the kit of Example 12 was used instead of the kit of Example 1 whereupon HDL cholesterol in 30 human serum samples was measured using Hitachi 7170 autoanalyzer.

### Example 33: Quantitative determination of HDL cholesterol

The same operation as in the measuring method of Example 21 was conducted except that the kit of Example 13 was used instead of the kit of Example 1 whereupon HDL cholesterol in 30 human serum samples was measured using Hitachi 7170 autoanalyzer.

### Example 34: Quantitative determination of HDL cholesterol

The same operation as in the measuring method of Example 21 was conducted except that the kit of Example 14 was used instead of the kit of Example 1 whereupon HDL cholesterol in 30 human serum samples was measured using Hitachi 7170 autoanalyzer.

### Example 35: Quantitative determination of HDL cholesterol

The same operation as in the measuring method of Example 21 was conducted except that the kit of Example 15 was used instead of the kit of Example 1 whereupon HDL cholesterol in 30 human serum samples was measured using Hitachi 7170 autoanalyzer.

### Example 36: Quantitative determination of HDL cholesterol

The same operation as in the measuring method of Example 21 was conducted except that the kit of Example 16 was used instead of the kit of Example 1 whereupon HDL cholesterol in 30 human serum samples was measured using Hitachi 7170 autoanalyzer.

### Comparative Example 7: Quantitative determination of HDL cholesterol

The same operation as in the measuring method of Example 21 was conducted except that the kit of Comparative Example 3 was used instead of the kit of Example 1 whereupon HDL cholesterol in 30 human serum samples was measured using Hitachi 7170 autoanalyzer.

### Comparative Example 8: Quantitative determination of HDL cholesterol

The same operation as in the measuring method of Example 21 was conducted except that the kit of Comparative Example 4 was used instead of the kit of Example 1 whereupon HDL cholesterol in 30 human serum samples was measured using Hitachi 7170 autoanalyzer.

### Example 37: Quantitative determination of HDL cholesterol

The same operation as in the measuring method of Example 21 was conducted except that the kit of Example 17 was used instead of the kit of Example 1 whereupon HDL cholesterol in 30 human serum samples was measured using Hitachi 7170 autoanalyzer.

### Example 38: Quantitative determination of HDL cholesterol

The same operation as in the measuring method of Example 21 was conducted except that the kit of Example 18 was used instead of the kit of Example 1 whereupon HDL cholesterol in 30 human serum samples was measured using Hitachi 7170 autoanalyzer.

### Example 39: Quantitative determination of HDL cholesterol

The same operation as in the measuring method of Example 21 was conducted except that the kit of Example 19 was used instead of the kit of Example 1 whereupon HDL cholesterol in 30 human serum samples was measured using Hitachi 7170 autoanalyzer.

### Example 40: Quantitative determination of HDL cholesterol

The same operation as in the measuring method of Example 21 was conducted except that the kit of Example 20 was used instead of the kit of Example 1 whereupon HDL cholesterol in 30 human serum samples was measured using Hitachi 7170 autoanalyzer.

In the meanwhile, HDL cholesterol in the 30 human serum samples used in the measurements of Examples 21 to 40 and Comparative Examples 5 to 8 was measured according to a DCM (a Designated Comparison Method) mentioned in *Clinical Chemistry,* vol. 45, no. 10 (1999) and compared with each measuring method.

Correlation coefficients between the measuring method mentioned in Examples 21 to 40 or Comparative Examples 5 to 8 and the measuring method by the DCM are shown in Table 1.

**Table 1**

| Method | Kit | Bile Acid Derivative | Cholesterol Esterase | BSA | Correlation Coefficient |
|---|---|---|---|---|---|
| Ex 21 | Ex 1 | Na cholate | LPL311 modified by VFM-4101 | - | 0.934 |
| Ex 22 | Ex 2 | Na cholate | LPL311 modified by VFM-4101 | + | 0.928 |
| Ex 23 | Ex 3 | Na taurocholate | LPL311 modified by VFM-4101 | - | 0.889 |
| Ex 24 | Ex 4 | Na taurocholate | LPL311 modified by VFM-4101 | + | 0.869 |
| Ex 25 | Ex 5 | BIGCHAP | LPL311 modified by VFM-4101 | - | 0.991 |
| Ex 26 | Ex 6 | BIGCHAP | LPL311 modified by VFM-4101 | + | 0.983 |
| Ex 27 | Ex 7 | CHAPS | LPL311 modified by VFM-4101 | - | 0.986 |
| Ex 28 | Ex 8 | CHAPS | LPL311 modified by VFM-4101 | + | 0.984 |
| CE 5 | CE 1 | - | LPL311 modified by VFM-4101 | - | 0.013 |
| CE 6 | CE2 | - | LPL311 modified by VFM-4101 | + | 0.008 |
| Ex 29 | Ex 9 | Na cholate | LPL6 (unmodified) | + | 0.981 |
| Ex 30 | Ex 10 | Na cholate | LPL6 (unmodified) | - | 0.774 |
| Ex 31 | Ex 11 | Na taurocholate | LPL6 (unmodified) | + | 0.983 |
| Ex 32 | Ex 12 | Na taurocholate | LPL6 (unmodified) | - | 0.726 |
| Ex 33 | Ex 13 | BIGCHAP | LPL6 (unmodified) | - | 0.968 |
| Ex 34 | Ex 14 | BIGCHAP | LPL6 (unmodified) | + | 0.991 |
| Ex 35 | Ex 15 | CHAPS | LPL6 (unmodified) | - | 0.977 |
| Ex 36 | Ex 16 | CHAPS | LPL6 (unmodified) | + | 0.994 |
| CE 7 | CE 3 | - | LPL6 (unmodified) | - | 0.414 |
| CE 8 | CE 4 | - | LPL6 (unmodified) | + | 0.462 |
| Ex 37 | Ex 17 | Na cholate | CHE2 modified by VFM-4101 | - | 0.948 |
| Ex 38 | Ex 18 | Na cholate | LPL311 modified by MEAC-50HS | - | 0.960 |
| Ex 39 | Ex 19 | Na cholate | LPL311 modified by AKM-1510 | - | 0.935 |
| Ex 40 | Ex 20 | Na cholate | COE313 | - | 0.916 |
| Ex: Example; | | | | | |
| CE: Comparative Example; | | | | | |
| Na cholate: sodium cholate; | | | | | |
| Na taurocholate: Sodium taurocholate | | | | | |

As clearly shown in the result of Examples 21 to 28, when chemically modified cholesterol esterase is used as cholesterol esterase, good correlation was noted with the determination by a DCM in the presence of any of cholic acid, taurocholic acid, BIGCHAP and CHAPS regardless of the presence or absence of albumin. However, as shown in Comparative Examples 5 and 6, a good correlation was not noted with the determination by a DCM in the absence of a bile acid derivative regardless of the presence or absence of albumin.

From comparisons of Example 29 with Example 30 , Example 31 with Example 32, Example 33 with Example 34 and Example 35 with Example 36, it is noted that, when an unmodified enzyme is used as cholesterol esterase and cholic acid or taurocholic acid is present as a bile acid derivative, a good correlation with the determination by a DCM is achieved by addition of albumin while under the condition where BIGCHAP or CHAPS is present as a bile acid derivative, a good correlation with the determination by a DCM is achieved regardless of the presence or absence of albumin.

From comparison of Example 21 with Example 37, it is noted that a good correlation with the determination by a DCM is achieved even when chemically modified cholesterol esterase other than chemically modified LPL311 is used. From comparisons of Example 21 with Example 38 and Example 21 with Example 39, it is noted that a good correlation to the determination by a DCM is achieved even when chemically modified cholesterol esterase which is prepared by the using a modifying agent other than VFM-4101. From comparison of Example 21 with Example 40, it is noted that a good correlation to the determination by a DCM is achieved even when a commercially available product is used as chemically modified cholesterol esterase.

### Example 41: Synthesis of a cholate [Compound (IIIa-1)]

Poly(ethylene glycol) methyl ether (average molecular weight: 2,000; manufactured by Aldrich) (110 g, 55 mmol), 6.7 g (55 mmol) of dimethylaminopyridine (manufactured by Koei Chemical) and 100 mL of dichloromethane were added to and dissolved in a flask equipped with a dropping funnel, a stirrer, a thermometer, a condenser and an introducing tube for nitrogen gas. To the resulting solution were added 20.4 g (50 mmol) of cholic acid (manufactured by Mikuni Chemical Industries) and then 10.5 g (55 mmol) of 1-ethyl-3-(3-dimethylaminopropyl)-carbodiimide hydrochloride (manufactured by Eiweiss Chemical). The mixture was stirred at room temperature for 12 hours under a nitrogen atmosphere and extracted with 200 mL of dichloromethane and 300 mL of water. An organic layer was washed with 200 mL of 0.2 mol/L hydrochloric acid and washed with 300 mL of 10% brine twice. After that, 500 mL of ethyl acetate was added to the separated organic layer followed by washing with 300 mL of 10% brine thrice. The organic layer was dried over magnesium sulfate, the drying agent was removed by filtration and the filtrate was concentrated using a rotary evaporator. It was further dried *in vacuo* at 80°C for 2 hours using a vacuum pump to give 115 g (yield: 96%) of Compound (IIIa-1) as white wax. 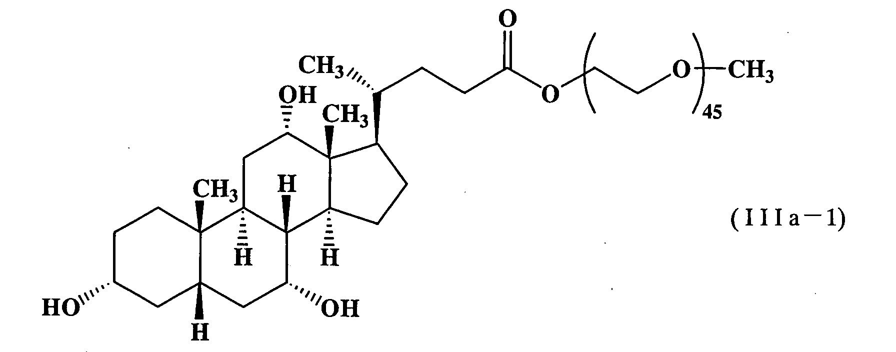 ¹H-NMR (CDCl₃, δ ppm, 400 MHz) : 0.69 (s, 3H), 0.90 (s, 3H), 0.98 (d, J = 6.0 Hz, 3H), 0.98-2.40 (m, 27H), 3.38 (s, 3H), 3.64 (bs, 179H), 3.97 (s, 2H), 4.22 (m, 2H). ¹³C-NMR (CDCl₃, δ ppm, 100 MHz): 12.5, 17.3, 22.5, 23.2, 26.4, 27.5, 28.3, 30.4, 30.8, 31.1, 34.7, 35.2, 35.3, 39.5, 39.6, 41.5, 41.7, 46.4, 47.0, 59.0, 63.4, 68.2, 69.1, 70.5, 70.9, 71.7, 71.9, 72.9, 174.2. IR (KBr Disk, cm⁻¹): 2887, 1734, 1468, 1343, 1149, 1111, 1060, 963, 842. GPC(average molecular weight): Mw 2390 (Mw/Mn 1.05).

### Example 42: Synthesis of cholate [Compound (IIIa-2)]

Poly(ethylene glycol) methyl ether (average molecular weight: 400; UNIOX M-400 manufactured by NOF) (4.8 g, 12 mmol), 1. 46 g (12 mmol) of dimethylaminopyridine (manufactured by Koei Chemical) and 30 mL of dichloromethane were added to and dissolved in a flask. To the resulting solution were added 4.08 g (10 mmol) of cholic acid (manufacturedbyMikuni Chemical Industries) and then 2.3 g (12 mmol) of 1-ethyl-3-(3-dimethylaminopropyl)-carbodiimide hydrochloride (manufactured by Eiweiss Chemical). The mixture was stirred at room temperature for 12 hours under a nitrogen atmosphere and extracted with 20 mL of dichloromethane and 50 mL of water. An organic layer was washed with 50 mL of 0.2 mol/L hydrochloric acid and washed with 50 mL of 10% brine twice. After that, 100 mL of ethyl acetate was added to the separated organic layer followed by washing with 100 mL of 10% brine thrice. The organic layer was dried over magnesium sulfate, the drying agent was removed by filtration and the filtrate was concentrated using a rotary evaporator. It was further dried *in vacuo* at 80°C for 2 hours using a vacuum pump to give 7.8 g (yield: 95%) of Compound (IIIa-2) as colorless oil. 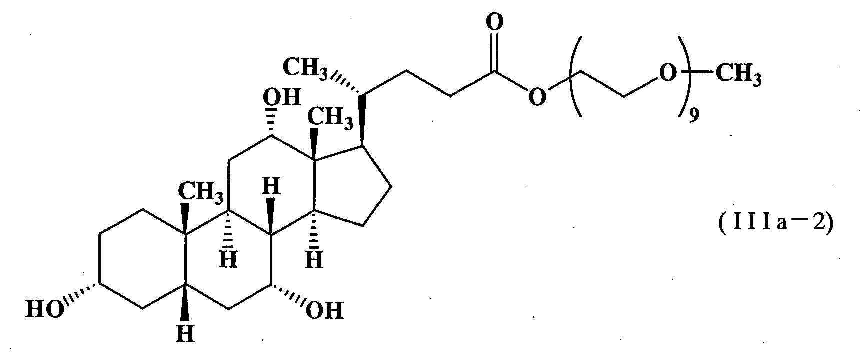 ¹H-NMR (CDCl₃, δ ppm, 400 MHz): 0.68 (s, 3H), 0.89 (s, 3H), 0.98 (d, J = 6.0 Hz, 3H), 0.98-2.31 (m, 27H), 3.38 (s, 3H), 3.45 (bs, 1H), 3.55 (bs, 2H), 3.65 (bs, 32H), 3.85 (bs, 1H), 3.96 (bs, 1H), 4.22 (bs, 2H). ¹³C-NMR (CDCl₃, δ ppm, 100 MHz): 12.5, 17.3, 22.5, 23.2, 26.3, 27.5, 28.2, 30.4, 30.9, 31.2, 34.7, 34.8, 35.3, 39.5, 41.5, 41.6, 46.4, 47.0, 59.0, 63.4, 68.4, 69.2, 70.6, 71.8, 71.9, 73.0, 174.3. IR (KBr Disk, cm⁻¹): 2931, 2868, 1734, 1451, 1375, 1299, 1251, 1110, 1111, 1045, 950, 856. GPC(average molecular weight): Mw 820 (Mw/Mn 1.04).

### Example 43: Synthesis of cholate [Compound (IIIa-3)]

Poly(ethylene glycol) methyl ether (average molecular weight: 1,000; UNIOX M-1000 manufactured by NOF) (12 g, 12 mmol), 1.46 g (12 mmol) of dimethylaminopyridine (manufactured by Koei Chemical) and 50 mL of dichloromethane were added to and dissolved in a flask. To the resulting solution were added 4.08 g (10 mmol) of cholic acid (manufacturedbyMikuni Chemical Industries) and then 2.3 g (12 mmol) of 1-ethyl-3-(3-dimethylaminopropyl)-carbodiimide hydrochloride (manufactured by Eiweiss Chemical). The mixture was stirred at room temperature for 12 hours under a nitrogen atmosphere and extracted with 30 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 200 mL of 10% brine thrice. The organic layer was dried over magnesium sulfate, the drying agent was removed by filtration and the filtrate was concentrated using a rotary evaporator. It was further dried *in vacuo* at 80°C for 2 hours using a vacuum pump to give 13.2 g (yield: 95%) of Compound (IIIa-3) as white wax. 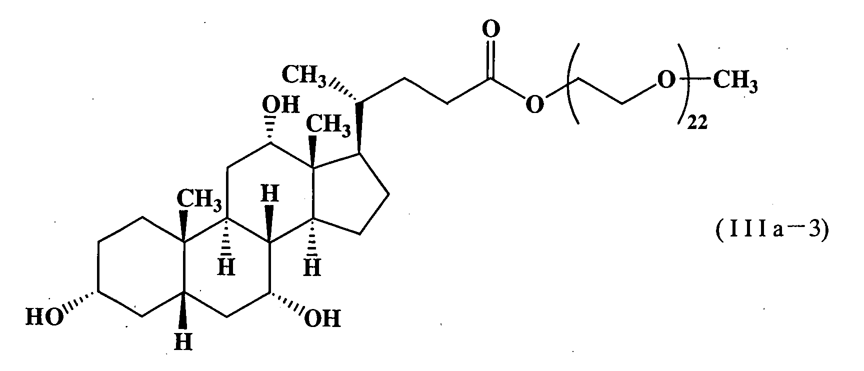 ¹H-NMR (CDCl₃, δ ppm, 400 MHz) : 0.68 (s, 3H), 0.89 (s, 3H), 0.98 (d, J = 6.2 Hz, 3H), 0.98-2.45 (m, 27H), 3.38 (s, 3H), 3.46 (bs, 1H), 3.55 (bs, 2H), 3.64 (bs, 84H), 3.84 (bs, 1H), 3.96 (bs, 1H), 4.22 (bs, 2H). ¹³C-NMR (CDCl₃, δ ppm, 100 MHz): 12. 5, 17.3, 22.5, 23.2, 26.4, 27.5, 28.3, 30.4, 30.8, 31.2, 34.8, 35.3, 39.5, 41.5, 41.7, 46.4, 47.0, 59.0, 63.4, 68.3, 69.1, 70.5, 70.9, 71.8, 71.9, 72.9, 174.2. IR (KBr Disk, cm⁻¹): 2870, 1732, 1466, 1345, 1112, 950, 844. GPC(average molecular weight): Mw 1430 (Mw/Mn 1.04).

### Example 44: Synthesis of a cholate [Compound (IIIa-4)]

Poly(ethylene glycol) methyl ether (average molecular weight: 5,000; manufactured by Aldrich) 55 g (11 mmol), 1.46 g (12 mmol) of dimethylaminopyridine (manufactured by Koei Chemical) and 100 mL of dichloromethane were added to and dissolved in a flask. To the resulting solution were added 4.08 g (10 mmol) of cholic acid (manufactured by Mikuni Chemical Industries) and then 2.3 g (12 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 300 mL of ethyl acetate was added to the separated organic layer followed by washing with 300 mL of 10% brine thrice. The organic layer was dried over magnesium sulfate, the drying agent was removed by filtration and the filtrate was concentrated using a rotary evaporator. It was further dried in *vacuo* at 80°C for 2 hours using a vacuum pump to give 48.5 g (yield: 90%) of Compound (IIIa-4) as white wax. 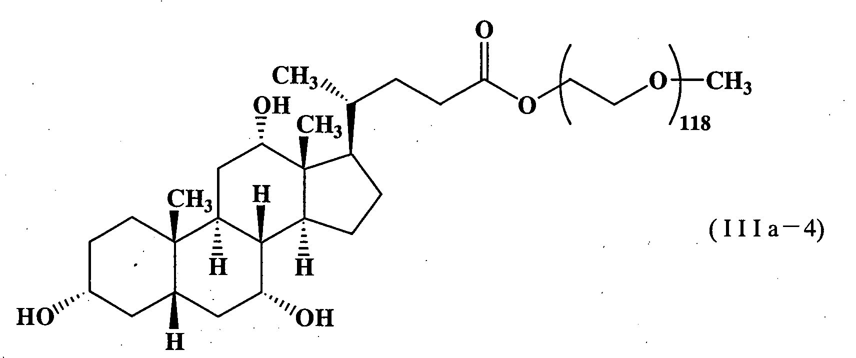 ¹H-NMR (CDCl₃, δ ppm, 400 MHz): 0.69 (s, 3H), 0.90 (s, 3H), 0.98 (d, J = 6.2 Hz, 3H), 0.98-2.43 (m, 27H), 3.45 (s, 3H), 3.56 (bs, 473H), 4.22 (bs, 2H). ¹³C-NMR (CDCl₃, δ ppm, 100 MHz) : 12.5, 17.2, 22.5, 23.1, 26.4, 27.4, 28.3, 30.4, 30.8, 31.0, 34.6, 34.7, 35.1, 35.3, 39.5, 39.6, 41.5, 41.6, 46.3, 46.8, 58.9, 59.0, 61.4, 63.3, 67.9, 69.0, 69.6, 70.2, 70.5, 70.7, 71.0, 71.3, 71.5, 71.8, 72.5, 174.0. IR (KBr Disk, cm⁻¹): 2888, 1734, 1468, 1343, 1281, 1112, 963, 842. GPC(average molecular weight): Mw 5370 (Mw/Mn 1.04).

### Example 45: Synthesis of deoxycholate [Compound (IIIa-5)]

Poly(ethylene glycol) methyl ether (average molecular weight: 2,000; manufactured by Aldrich) (22 g, 11 mmol), 1.34 g (11 mmol) of dimethylaminopyridine (manufactured by Koei Chemical) and 50 mL of dichloromethane were added to and dissolved in a flask. To the resulting solution were added 3.93 g (10 mmol) of deoxycholic acid and then 2.3 g (12 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 300 mL of 10% brine thrice. The organic layer was dried over magnesium sulfate, the drying agent was removed by filtration and the filtrate was concentrated using a rotary evaporator. It was further dried *in vacuo* at 80°C for 2 hours using a vacuum pump to give 24.2 g (yield: 99%) of Compound (IIIa-5) as white wax. 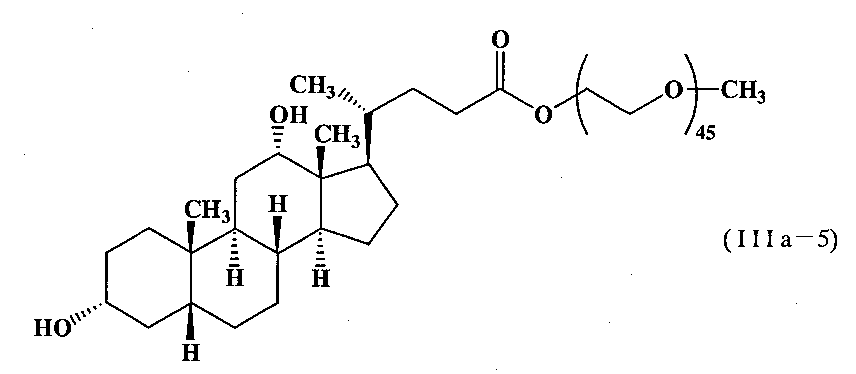 ¹H-NMR (CDCl₃, δ ppm, 400 MHz): 0.68 (s, 3H), 0.91 (s, 3H), 0.98 (d, J = 6.3 Hz, 3H), 0.98-2.45 (m, 28H), 3.38 (s, 3H), 3.47 (bs, 1H), 3.65 (bs, 178H), 3.80 (bs, 1H), 4.22 (bs, 2H). ¹³C-NMR (CDCl₃, δ ppm, 100 MHz) : 12.7, 17.3, 23.2, 26.1, 27.1, 27.4, 28.7, 30.4, 30.8, 31.1, 33.6, 34.1, 35.1, 35.3, 36.0, 37.0, 42.0, 46.4, 47.2, 48.2, 59.0, 61.6, 63.4, 69.1, 70.3, 70.5, 70.9, 71.2, 71.9, 72.6, 72.9, 174.1. IR (KBr Disk, cm⁻¹): 2889, 1735, 1468, 1343, 1281, 1112, 963, 842. GPC(average molecular weight): Mw 2510 (Mw/Mn 1.08).

### Example 46: Synthesis of ursodeoxycholate [Compound (IIIa-6)]

Poly(ethylene glycol) methyl ether (average molecular weight: 2,000; manufactured by Aldrich) (22 g, 11 mmol), 1.34 g (11 mmol) of dimethylaminopyridine (manufactured by Koei Chemical) and 50 mL of dichloromethane were added to and dissolved in a flask. To the resulting solution were added 3.93 g (10 mmol) of ursodeoxycholic acid and then 2.3 g (12 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 300 mL of 10% brine thrice. The organic layer was dried over magnesium sulfate, the drying agent was removed by filtration and the filtrate was concentrated. It was further dried *in vacuo* at 80°C for 2 hours using a vacuum pump to give 23.7 g (yield: 99%) of Compound (IIIa-6) as white wax. 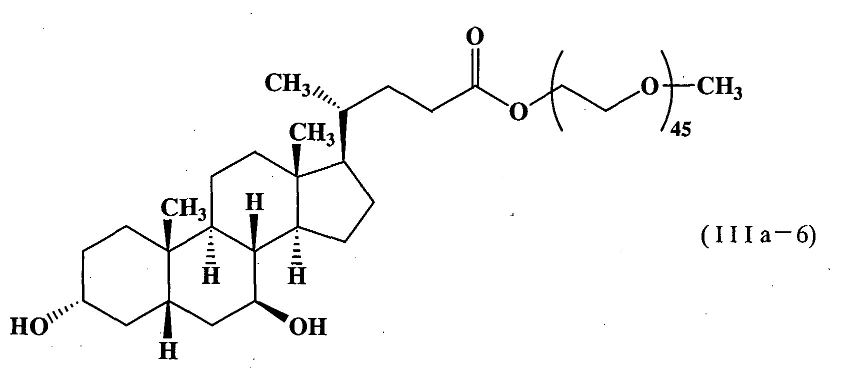 ¹H-NMR (CDCl₃, δ ppm, 400 MHz) : 0.68 (s, 3H), 0.92 (d, J = 6.3 Hz, 3H), 0.95(s, 3H), 0.96-2.45 (m, 28H), 3.38 (s, 3H), 3.65 (bs, 180H), 4.22 (bs, 2H). ¹³C-NMR (CDCl₃, δ ppm, 100 MHz) : 12.1, 18.4, 23.4, 26.9, 28.5, 30.3, 30.9, 31.1, 34.0, 35.0, 35.2, 37.1, 37.3, 39.1, 40.1, 42.4, 43.7, 54.9, 55.8, 59.0, 61.6, 63.3, 69.1, 70.3, 70.5, 70.8, 71.0, 71.1, 71.9, 72.5, 174.0. IR (KBr Disk, cm⁻¹): 2889, 1736, 1468, 1343, 1281, 1112, 963, 842. GPC (average molecular weight): Mw 2530 (Mw/Mn 1.08).

### Example 47: Synthesis of chenodeoxycholate [Compound (IIIa-7)]

Poly(ethylene glycol) methyl ether (average molecular weight: 2,000; manufactured by Aldrich) (22 g, 11 mmol), 1.34 g (11 mmol) of dimethylaminopyridine (manufactured by Koei Chemical) and 50 mL of dichloromethane were added to and dissolved in a flask. To the resulting solution were added 3.93 g (10 mmol) of chenodeoxycholic acid and then 2.3 g (12 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 300 mL of 10% brine thrice. The organic layer was dried over magnesium sulfate, the drying agent was removed by filtration and the filtrate was concentrated. It was further dried *in vacuo* at 80°C for 2 hours using a vacuum pump to give 23.2 g (yield: 97%) of Compound (IIIa-7) as white wax. 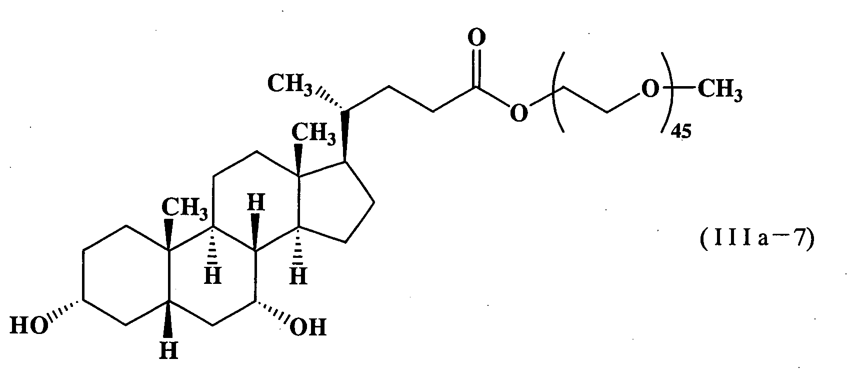 ¹H-NMR (CDCl₃,δ ppm, 400 MHz) : 0.66 (s, 3H), 0.91(s, 3H), 0.92 (d, J = 6.3 Hz, 3H), 0.92-2.43 (m, 28H), 3.38 (s, 3H), 3.64 (bs, 180H), 4.22 (bs, 2H). ¹³C-NMR (CDCl₃, δ ppm, 100 MHz): 11.8, 18.3, 22.8, 23.6, 28.1, 30.6, 30.9, 31.0, 32.8, 34.7, 35.0, 35.3, 35.4, 39.4, 39.6, 39.8, 41.5, 42.6, 50.4, 55.7, 59.0, 61.6, 63.3, 68.2, 69.1, 69.6, 70.3, 70.5, 70.8, 71.4, 71.7, 71.9, 72.6, 174.1. IR (KBr Disk, cm⁻¹): 2889, 1736, 1468, 1343, 1281, 1111, 963, 842. GPC (average molecular weight): Mw 2560 (Mw/Mn 1.09).

### Example 48: Synthesis of lithocholate [Compound (IIIa-8)]

Poly(ethylene glycol) methyl ether (average molecular weight: 2,000; manufactured by Aldrich) (22 g, 11 mmol), 1.34 g (11 mmol) of dimethylaminopyridine (manufactured by Koei Chemical) and 50 mL of dichloromethane were added to and dissolved in a flask. To the resulting solution were added 3.77 g (10 mmol) of lithocholic acid and then 2.3 g (12 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 300 mL of 10% brine thrice. The organic layer was dried over magnesium sulfate, the drying agent was removed by filtration and the filtrate was concentrated. It was further dried *in vacuo* at 80°C for 2 hours using a vacuum pump to give 23.3 g (yield: 98%) of Compound (IIIa-8) as white wax. 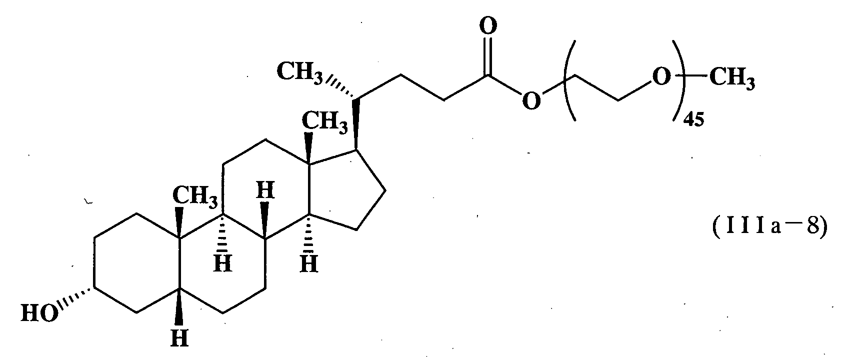 ¹H-NMR (CDCl₃, δ ppm, 400 MHz): 0.64 (s, 3H), 0.91 (d, J = 6.3 Hz, 3H), 0.92(s, 3H), 0.92-2.41 (m, 29H), 3.38 (s, 3H), 3.64 (bs, 179H), 4.22 (bs, 2H). ¹³C-NMR (CDCl₃, δ ppm, 100 MHz) : 12.0, 18.3, 20.8, 23.4, 24.2, 26.4, 27.2, 28.1, 30.5, 30.9, 31.1, 34.5, 35.3, 35.4, 35.8, 36.4, 40.1, 40.4, 42.0, 42.7, 55.9, 56.4, 59.0, 61.6, 63.3, 69.1, 69.7, 70.3, 70.5, 70.6, 70.7, 70.8, 71.1, 71.5, 71.9, 72.6, 174.1. IR (KBr Disk, cm⁻¹): 2888, 1736, 1468, 1343, 1281, 1111, 963, 842. GPC(average molecular weight): Mw 2640 (Mw/Mn 1.06).

### Example 49: Synthesis of lithocholate where both terminals are esterified [Compound (IIIb-1)]

Poly(ethylene glycol) (PEG 2000; manufactured by Dai-ichi Kogyo Seiyaku) (10 g, 5 mmol), 2.68 g (22 mmol) of dimethylaminopyridine (manufactured by Koei Chemical) and 50 mL of dichloromethane were added to and dissolved in a flask. To the resulting solution were added 4.08 g (10 mmol) of cholic acid and then 4.6 g (24 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 300 mL of 10% brine thrice. The organic layer was dried over magnesium sulfate, the drying agent was removed by filtration and the filtrate was concentrated. It was further dried *in vacuo* at 80°C for 2 hours using a vacuum pump to give 13.2 g (yield: 94%) of Compound (IIIb-1) as white wax. 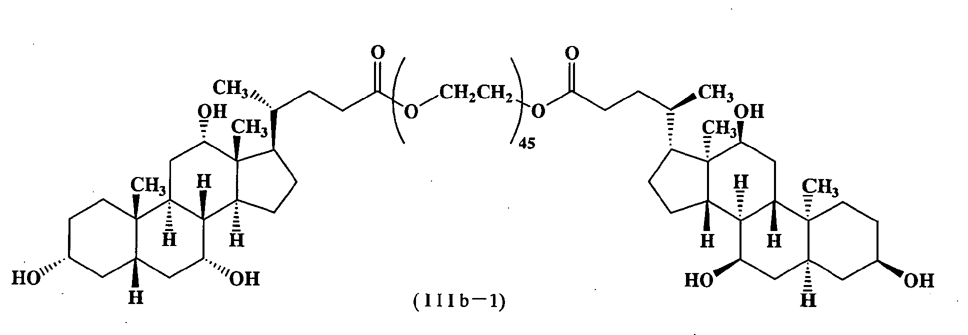 ¹H-NMR (CDCl₃, δ ppm, 400MHz): 0.68 (s, 6H), 0.90 (s, 6H), 0.99(d, J = 6.1 Hz, 6H), 0.92-2.67 (m, 54H), 3.65 (bs, 180H), 3.96 (bs, 2H), 4.22 (bs, 4H). ¹³C-NMR (CDCl₃, δ ppm, 100 MHz): 12.5, 17.3, 22.4, 23.2, 26.3, 27.5, 28.2, 30.3, 30.8, 31.2, 34.7, 35.3, 39.5, 41.5, 41.6, 46.4, 46.5, 46.9, 61.6, 63.4, 68.3, 69.1, 69.7, 70.2, 70.4, 70.5, 70.6, 70.8, 71.3, 71.7, 72.6, 72.9, 174.2. IR (KBr Disk, cm⁻¹): 2885, 1734, 1468, 1345, 1281, 1113, 964, 842. GPC(average molecular weight): Mw 2940 (Mw/Mn 1.05).

### Example 50: Synthesis of cholate [Compound (IIIa-9)]

Poly(ethylene glycol) (PEG 600; manufactured by Dai-ichi Kogyo Seiyaku) (12 g, 20 mmol), 1.5 g (12 mmol) of dimethylaminopyridine (manufactured by Koei Chemical) and 50 mL of dichloromethane were added to and dissolved in a flask. To the resulting solution were added 4.08 g (10 mmol) of cholic acid and then 2.3 g (12 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 300 mL of 10% brine thrice. The organic layer was dried over magnesium sulfate, the drying agent was removed by filtration and the filtrate was concentrated. It was further dried *in vacuo* at 80°C for 2 hours using a vacuum pump to give 7.7 g (yield: 90%) of Compound (IIIa-9) as white wax. 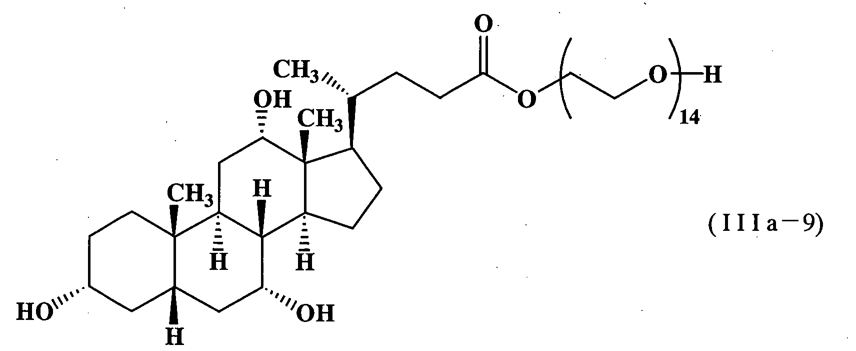 ¹H-NMR (CDCl₃, δ ppm, 400MHz): 0.68 (s, 3H), 0.90 (s, 3H), 0.98(d, J = 6.1 Hz, 3H), 0.92-2.50 (m, 27H), 3.15 (bs, 1H), 3.44 (bs, 1H), 3.66 (bs, 54H), 3.84 (bs, 1H), 3.96 (bs, 1H), 4.23 (bs, 2H). ¹³C-NMR (CDCl₃, δ ppm, 100 MHz): 12.5, 17.3, 22.5, 23.2, 26.3, 27.5, 28.2, 30.3, 30.8, 31.2, 34.7, 34.8, 35.3, 35.4, 39.5, 41.5, 46.4, 47.0, 61.5, 63.4, 68.3, 69.1, 69.7, 70.2, 70.5, 71.8, 72.6, 73.0, 174.3. IR (KBr Disk, cm⁻¹): 3404, 2929, 2868, 1732, 1453, 1352, 1251, 1103, 951. GPC(average molecular weight): Mw 1050 (Mw/Mn 1.05).

### Example 51: Synthesis of cholic acid amide [Compound (IIIb-2)]

Polyoxyethylenediamine (average molecular weight: 2000; PEO Amine #2000 manufactured by Kawaken Fine Chemicals) (10 g, 5 mmol), 2.56 g (21 mmol) of 4-dimethylaminopyridine (manufactured by Koei Chemical) and 50 mL of dichloromethane were added to and dissolved in a flask. To the resulting solution were added 4.08 g (10 mmol) of cholic acid (manufactured by Mikuni Chemical Industries) and then 4.6 g (24 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. An organic layer was washed with 50 mL of 0.2 mol/L hydrochloric acid and washed with 50 mL of 10% brine twice. After that, 200 mL of ethyl acetate was added to the separated organic layer followed by washing with 300 mL of 10% brine thrice. The organic layer was dried over magnesium sulfate, the drying agent was removed by filtration and the filtrate was concentrated. It was further dried *in vacuo* at 80°C for 2 hours using a vacuum pump to give 12.4 g (yield: 88%) of Compound (IIIb-2) as white wax. 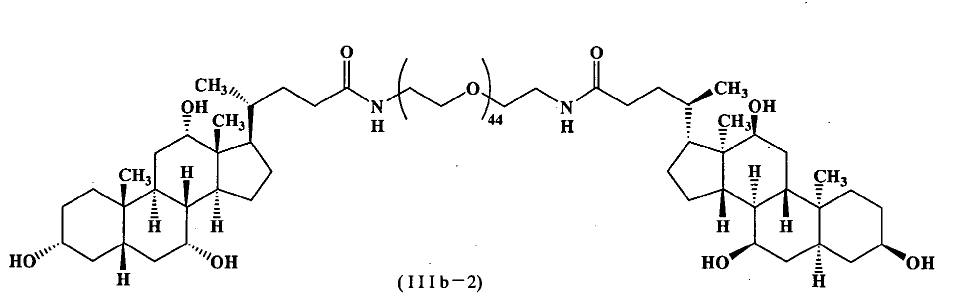 ¹H-NMR (CDCl₃, δ ppm, 400 MHz): 0.68 (s, 6H), 0.89 (s, 6H), 0.99(d, J = 6.1 Hz, 6H), 1.12-2.28 (m, 48H), 2.72 (bs, 6H), 3.31 (bs, 2H), 3.34 (bs, 4H), 3.65 (m, 176H), 3.96 (bs, 4H), 4.22 (bs, 2H). ¹³C-NMR (CDCl₃, δ ppm, 100 MHz): 12.5, 17.4, 22.5, 23.3, 26.3, 27.6, 28.1, 29.1, 30.3, 31.7, 33.2, 34.8, 35.4, 37.5, 39.4, 41.5, 46.3, 46.5, 68.3, 69.7, 70.1, 70.2, 70.5, 70.8, 71.7, 73.0, 174.1. IR (KBrDisk, cm⁻¹): 3442, 2912, 2871, 1648, 1456, 1352, 1251, 1107, 952. GPC(average molecular weight): Mw 2990(Mw/Mn 1.07).

### Example 52: Synthesis of cholic acid amide [Compound (IIIa-10)]

Polyoxyethylenediamine (average molecular weight: 1000; PEO Amine #1000 manufactured by Kawaken Fine Chemicals) (15 g, 15 mmol), 1.46 g (12 mmol) of 4-dimethylaminopyridine (manufactured by Koei Chemical) and 50 mL of dichloromethane were added to and dissolved in a flask. To the resulting solution were added 4.08 g (10 mmol) of cholic acid (manufactured by Mikuni Chemical Industries) and then 2.3 g (12 mmol) of 1-ethyl-3-(3-dimethylaminopropyl)carbodiimide hydrochloride (manufactured by Eiweiss Chemical). The mixture was stirred at room temperature for 12 hours under a nitrogen atmosphere and extracted with 50 mL of dichloromethane and 50 mL of water. The separated organic layer was washed with 22 mL of 1 mol/L hydrochloric acid and 50 mL of water and further washed with 50 mL of 10% brine twice. The organic layer was dried over magnesium sulfate, the drying agent was removed by filtration and the filtrate was concentrated using rotary evaporator. It was further dried *in vacuo* at 80°C for 2 hours using a vacuum pump to give 11. 9 g (yield: 86%) of Compound (IIIa-10) as light yellow viscous liquid. 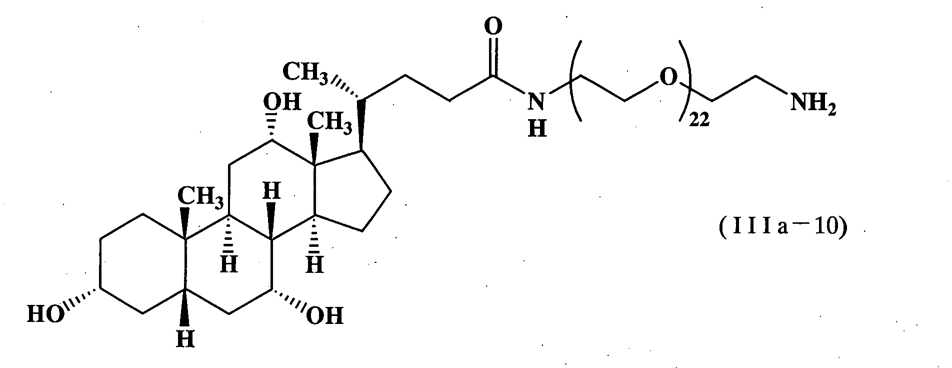 ¹H-NMR (CDCl₃, δ ppm, 400 MHz): 0.68 (s, 3H), 0.89 (s, 3H), 0.99 (d, J = 7.7 Hz, 3H), 0.98-2.28 (m, 24H), 2.89 (bs, 3H), 3.29 (bt, J = 6.0 Hz , 2H), 3.31 (bt, J = 6.0 Hz , 2H), 3.53 (bs, 1H), 3.64 (bs, 84H), 3.83 (bs, 2H), 3.96 (bs, 2H), 4.22 (s, 2H), 7.28 (bs, 1H), 7.85 (bs, 2H). ¹³C-NMR (CDCl₃, δ ppm, 100 MHz) : 12.5, 17.3, 17.4, 22.4, 23.3, 26.3, 26.5, 27.6, 28.1, 29.1, 30.3, 31.7, 33.1, 34.7, 34.8, 35.4, 37.3, 39.2, 39.5, 41.6, 46.3, 46.5, 68.2, 69.3, 69.4, 69.6, 70.0, 70.1, 70.2, 70.3, 70.4, , 70.8, 71.7, 73.0, 174.1. IR (KBr Neat, cm⁻¹): 3372, 2867, 1648, 1464, 1350, 1298, 1251, 1110, 950. GPC(average molecular weight): Mw 1400 (Mw/Mn 1.06).

### Example 53: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIa-1) | 6.0 g/L |

### Example 54: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| Compound (IIIa-2) | 0.15 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |

### Example 55: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIa-3) | 1.5 g/L |

### Example 56: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIb-2) | 1.0 g/L |

### Example 57: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| Compound (IIIa-5) | 0.5 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |

### Example 58: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIa-1) | 6.0 g/L |

### Example 59: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| Sodium dextran sulfate (molecular weight: 500,000) | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIa-1) | 6.0 g/L |

### Example 60: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| Sodium dextran sulfate (molecular weight: 500,000) | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIb-2) | 1.0 g/L |

### Example 61: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| Sodium dextran sulfate (molecular weight: 500,000) | 1.0 g/L |
| Compound (IIIa-2) | 0.15 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |

### Example 62: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| Sodium dextran sulfate (molecular weight: 500,000) | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIa-3) | 1.5 g/L |

### Example 63: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |
| Sodium dextran sulfate (molecular weight: 500,000) | |
| | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIa-1) | 6.0 g/L |

### Example 64: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |
| Sodium dextran sulfate (molecular weight: 500,000) | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIb-2) | 1.0 g/L |

### Example 65: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |
| Sodium dextran sulfate (molecular weight: 500,000) | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |
| Compound (IIIa-3) | 1.5 g/L |

### Comparative Example 9: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |

### Comparative Example 10: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |

### Comparative Example 11: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| Sodium dextran sulfate (molecular weight: 500,000) | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |

### Comparative Example 12: Kit for quantitatively determining HDL cholesterol

A kit for quantitatively determining HDL cholesterol comprising the following first reagent and second reagent was prepared.

| First Reagent | |
|---|---|
| HEPES (pH 7.5) | 10 mmol/L |
| EMSE | 0.3 g/L |
| BSA | 2.0 g/L |
| Sodium dextran sulfate (molecular weight: 500,000) | 1.0 g/L |

| Second Reagent | |
|---|---|
| HEPES (pH 7.0) | 10 mmol/L |
| 4-Aminoantipyrine | 0.3 g/L |
| Peroxidase | 20 kU/L |
| Chemically modified LPL311 (modified by VFM-4101) | 0.2 kU/L |
| COO321 | 5.0 kU/L |

### Example 66: Quantitative determination of HDL cholesterol

The same operation as in the measuring method of Example 21 was conducted except that the kit of Example 53 was used instead of the kit of Example 1 whereupon HDL cholesterol in 35 human serum samples was measured using Hitachi 7170 autoanalyzer.

### Example 67: Quantitative determinin of HDL cholesterol

The same operation as in the measuring method of Example 21 was conducted except the kit of Example 54 was used instead of the kit of Example 1 whereupon HDL cholesterol in 35 human serum samples was measured using Hitachi 7170 autoanalyzer.

### Example 68: Quantitative determination of HDL cholesterol

The same operation as in the measuring method of Example 21 was conducted except that the kit of Example 55 was used instead of the kit of Example 1 whereupon HDL cholesterol in 35 human serum samples was measured using Hitachi 7170 autoanalyzer.

### Example 69: Quantitative determination of HDL cholesterol

The same operation as in the measuring method of Example 21 was conducted except the kit of Example 56 was used instead of the kit of Example 1 whereupon HDL cholesterol in 35 human serum samples was measured using Hitachi 7170 autoanalyzer.

### Example 70: Quantitative determination of HDL cholesterol

The same operation as in the measuring method of Example 21 was conducted except that the kit of Example 57 was used instead of the kit of Example 1 whereupon HDL cholesterol in 35 human serum samples was measured using Hitachi 7170 autoanalyzer.

### Example 71: Quantitative determination of HDL cholesterol

The same operation as in the measuring method of Example 21 was conducted except that the kit of Example 58 was used instead of the kit of Example 1 whereupon HDL cholesterol in 35 human serum samples was measured using Hitachi 7170 autoanalyzer.

### Example 72: Quantitative determination of HDL cholesterol

The same operation as in the measuring method of Example 21 was conducted except that the kit of Example 59 was used instead of the kit of Example 1 whereupon HDL cholesterol in 35 human serum samples was measured using Hitachi 7170 autoanalyzer.

### Example 73: Quantitative determination of HDL cholesterol

The same operation as in the measuring method of Example 21 was conducted except that the kit of Example 60 was used instead of the kit of Example 1 whereupon HDL cholesterol in 35 human serum samples was measured using Hitachi 7170 autoanalyzer.

### Example 74: Quantitative determination of HDL cholesterol

The same operation as in the measuring method of Example 21 was conducted except that the kit of Example 61 was used instead of the kit of Example 1 whereupon HDL cholesterol in 35 human serum samples was measured using Hitachi 7170 autoanalyzer.

### Example 75: Quantitative determination of HDL cholesterol

The same operation as in the measuring method of Example 21 was conducted except that the kit of Example 62 was used instead of the kit of Example 1 whereupon HDL cholesterol in 35 human serum samples was measured using Hitachi 7170 autoanalyzer.

### Example 76: Quantitative determination of HDL cholesterol

The same operation as in the measuring method of Example 21 was conducted except that the kit of Example 63 was used instead of the kit of Example 1 whereupon HDL cholesterol in 35 human serum samples was measured using Hitachi 7170 autoanalyzer.

### Example 77: Quantitative determination of HDL cholesterol

The same operation as in the measuring method of Example 21 was conducted except that the kit of Example 64 was used instead of the kit of Example 1 whereupon HDL cholesterol in 35 human serum samples was measured using Hitachi 7170 autoanalyzer.

### Example 78: Quantitative determination of HDL cholesterol

The same operation as in the measuring method of Example 21 was conducted except that the kit of Example 65 was used instead of the kit of Example 1 whereupon HDL cholesterol in 35 human serum samples was measured using Hitachi 7170 autoanalyzer.

### Comparative Example 13: Quantitative determination of HDL cholesterol

The same operation as in the measuring method of Example 21 was conducted except that the kit of Comparative Example 9 was used instead of the kit of Example 1 whereupon HDL cholesterol in 35 human serum samples was measured using Hitachi 7170 autoanalyzer.

### Comparative Example 14: Quantitative determination of HDL cholesterol

The same operation as in the measuring method of Example 21 was conducted except that the kit of Comparative Example 10 was used instead of the kit of Example 1 whereupon HDL cholesterol in 35 human serum samples was measured using Hitachi 7170 autoanalyzer.

### Comparative Example 15: Quantitative determination of HDL cholesterol

The same operation as in the measuring method of Example 21 was conducted except that the kit of Comparative Example 11 was used instead of the kit of Example 1 whereupon HDL cholesterol in 35 human serum samples was measured using Hitachi 7170 autoanalyzer.

### Comparative Example 16: Quantitative determination of HDL cholesterol

The same operation as in the measuring method of Example 21 was conducted except that the kit of Comparative Example 12 was used instead of the kit of Example 1 whereupon HDL cholesterol in 35 human serum samples was measured using Hitachi 7170 autoanalyzer. In the meanwhile, HDL cholesterol in the 35 human serum samples used in Examples 66 to 78 and Comparative Examples 13 to 16 was measured according to a DCM and compared with each measuring method. Correlation coefficients between the measuring method mentioned in Examples 66 to 78 and Comparative Examples 13 to 16 and the measuring method by the DCM are shown in Table 2.

**Table 2**

| Method | Kit | Bile Acid Derivative | BSA | Dextran sulfate | Correlation Coefficient |
|---|---|---|---|---|---|
| Ex 66 | Ex 53 | Compound (IIIa-1) | - | - | 0.983 |
| Ex 67 | Ex 54 | Compound (IIIa-2) | - | - | 0.882 |
| Ex 68 | Ex 55 | Compound (IIIa-3) | - | - | 0.967 |
| Ex 69 | Ex 56 | Compound (IIIb-2) | - | - | 0.984 |
| Ex 70 | Ex 57 | Compound (IIIa-5) | - | - | 0.985 |
| Ex 71 | Ex 58 | Compound (IIIa-1) | + | - | 0.989 |
| Ex 72 | Ex 59 | Compound (IIIa-1) | - | + | 0.987 |
| Ex 73 | Ex 60 | Compound (IIIb-2) | - | + | 0.996 |
| Ex 74 | Ex 61 | Compound (IIIa-2) | - | + | 0.962 |
| Ex 75 | Ex 62 | Compound (IIIa-3) | - | + | 0.977 |
| Ex 76 | Ex 63 | Compound (IIIa-1) | + | + | 0.990 |
| Ex 77 | Ex 64 | Compound (IIIb-2) | + | + | 0.991 |
| Ex 78 | Ex 65 | Compound (IIIa-3) | + | + | 0.990 |
| CE 13 | CE 9 | | - | - | 0.020 |
| CE 14 | CE 10 | | + | - | 0.429 |
| CE 15 | CE 11 | | - | + | 0.006 |
| CE 16 | CE 12 | | + | + | 0.451 |
| Ex: Example; CE: Comparative Example | | | | | |

From Examples 66 to 70 and Comparative Example 13, a good correlation to the determination by the DCM was achieved when a bile acid ester or a bile acid amide was used as a bile acid derivative. From comparisons of Example 71 with Comparative Example 14, Examples 72 to 75 with Comparative Example 15 and Examples 76 to 78 with Comparative Example 16, it was noted that a good correlation coefficient to the determination by the DCM was achieved when a bile acid ester or a bile acid amide was used as a bile acid derivative even under the condition where BSA and/or dextran sulfate were added or where both compounds were added.

### Test Example 1: Influence of various bile acid derivatives (an anionic surfactant, a amphoteric surfactant and a nonionic surfactant) on the stability of enzymes for the quantitative determination of cholesterol

Reagents comprising the following compositions 1 to 8 used as the second reagent for the kit for quantitatively determining HDL cholesterol were prepared and each of them was stored at 40°C for 48 hours (two days) and 120 hours (five days). Each enzyme activity of cholesterol esterase (LPL311) and cholesterol oxidase (COO321) in each reagent stored at 40°C for 2 days and each reagent stored at 40°C for 5 days was measured and the percentage of the enzyme activity (residual percentage of enzyme activity) (%) in each reagent after storing to the enzyme activity in each reagent just after preparation was determined.

Enzyme activity was determined by the following method.

### (1) Method for the quantitative determination of enzyme activity of cholesterol esterase

| Reagent for the determination | |
|---|---|
| 0.15 mol/L potassium phosphate buffer (pH 7.2) | |
| Triton X-100 | 2 g/L |
| Phenol | 0.15 g/L |
| Peroxidase | 20 kU/L |
| Cholesterol oxidase | 5 kU/L |
| 4-Aminoantipyrine | 0.2 g/L |

### Substrate solution for the determination

Solution of cholesterol linoleate in isopropanol (6 g/L)

### Operation method

The above-identified reagent for the determination(3 mL) was added to quartz cells and 0.05 mL of each of 8 freshly prepared reagent having Compositions 1 to 8 respectively were added. After warming at 37°C for 5 minutes, 0.1 mL of a substrate solution for the determination was added to the reaction solution and the mixture was warmed at 37°C. Absorbance at 500 nm of each of the reaction solutions after 5 minutes and 6 minutes after the addition of the substrate solution for the determination was measured. Based on both absorbances, change in absorbance per minute (ΔAbs/min) at 500 nm for each of reagent having Compositions 1 to 8 was calculated. In the meanwhile, the same method was conducted using pure water instead of each of reagent having Compositions 1 to 8 and change in absorbance per minute (ΔAbs/min) at 500 nm for pure water was calculated. The change in absorbance per minute (ΔAbs/min) at 500 nm in pure water was subtracted from the change in absorbance per minute (ΔAbs/min) at 500 nm for each of reagent having Compositions 1 to 8 and the calculated value (ΔΔAbs/min) was used as an index for stabilization of cholesterol esterase.

A series of the same operations was conducted for the determination using each of the reagents where each of reagent having the compositions 1 to 8 was stored at 40°C for 2 days and using each of the reagents where each of reagent having the compositions 1 to 8 was stored at 40°C for 5 days instead of using each of freshly prepared reagents having compositions 1 to 8 and change in the absorbance (ΔΔAbs/min) for each reagent was calculated.

### (2) Method for the quantitative determination of enzyme activity of cholesterol oxidase

| Reagent for the determination | |
|---|---|
| 0.15 mol/L potassium phosphate buffer (pH 7.2) | |
| Triton X-100 | 2 g/L |
| Phenol | 0.15 g/L |
| Peroxidase | 20 kU/L |
| 4-Aminoantipyrine | 0.2 g/L |

### Substrate solution for the determination

Solution of cholesterol in isopropanol (5 g/L)

### Operation method

The above-identified reagent for the determination (3 mL) was added to quartz cells and 0.05 mL of each of the solution, prepared by 6-fold dilution with pure water of each of 8 freshly prepared reagent having Compositions 1 to 8 was added thereto. After warming at 37°C for 5 minutes, 0.1 mL of a substrate solution for the determination was added to the reaction solution and the mixture was warmed at 37°C. Absorbance at 500 nm of each of the reaction solutions after 5 minutes and 6 minutes after the addition of the substrate solution for the determination was measured. Based on both absorbances, change in absorbance per minute (ΔAbs/min) at 500 nm for each of reagent having Compositions 1 to 8 was calculated. In the meanwhile, the same method was conducted using pure water instead of each of reagent having Compositions 1 to 8 and change in absorbance per minute (ΔAbs/min) at 500 nm for pure water was calculated. The change in absorbance per minute (ΔAbs/min) at 500 nm for pure water was subtracted from the change in absorbance per minute (ΔAbs/min) at 500 nm for each of reagent having Compositions 1 to 8 and the calculated value (ΔΔAbs/min) was used as an index for stabilization of cholesterol oxidase.

A series of the same operations was conducted for the determination using each of the reagents where each reagent having Compositions 1 to 8 was stored at 40°C for 2 days and using each of the reagents where each reagent having Compositions 1 to 8 was stored at 40°C for 5 days instead of using each of the freshly prepared reagents having Compositions 1 to 8 and change in the absorbance (ΔΔAbs/min) for each reagent was calculated.

| Composition 1 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |

| Composition 2 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |
| Sodium cholate | 6.0 g/L |

| Composition 3 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |
| CHAPS | 6.0 g/L |

| Composition 4 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |
| BIGCHAP | 4.5 g/L |

| Composition 5 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |
| Compound (IIIa-1) | 6.0 g/L |

| Composition 6 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |
| Compound (IIIa-2) | 0.45 g/L |

| Composition 7 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |
| Compound (IIIa-3) | 4.5 g/L |

| Composition 8 | |
|---|---|
| HEPES (pH 7.0) | 10 mmol/L |
| 4-Aminoantipyrine | 0.3 g/L |
| Peroxidase | 20 kU/L |
| Calcium chloride dihydrate | 0.1 g/L |
| Chemically modified LPL311 (modified by VFM 4101) | 0.2 kU/L |
| Compound (IIIb-2) | 1.0 g/L |

Result of the determination is shown in Table 3.

**Table 3**

| Composition | Bile Acid Derivative | Enzyme | Residual Rate of Enzymatic Activity (%) | |
|---|---|---|---|---|
| | | | at 40°C for 2 Days | at 40°C for 5 Days |
| 1 | Nothing added | LPL311 | 95.1 | 92.3 |
| 2 | Cholic acid | LPL311 | 81.6 | 73.9 |
| 3 | CHAPS | LPL311 | 72.3 | 59.6 |
| 4 | BIGCHAP | LPL311 | 90.1 | 82.4 |
| 5 | Compound (IIIa-1) | LPL311 | 91.4 | 82.3 |
| 6 | Compound (IIIa-2) | LPL311 | 94.4 | 89.1 |
| 7 | Compound (IIIa-3) | LPL311 | 93.6 | 85.3 |
| 8 | Compound (IIIb-2) | LPL311 | 93.2 | 86.7 |
| 1 | Nothing added | COO321 | 100.3 | 100.0 |
| 2 | Cholic acid | COO321 | 0 | 0 |
| 3 | CHAPS | COO321 | 79.8 | 62.0 |
| 4 | BIGCHAP | COO321 | 100.0 | 92.3 |
| 5 | Compound (IIIa-1) | COO321 | 94.9 | 81.5 |
| 6 | Compound (IIIa-2) | C00321 | 100.7 | 96.3 |
| 7 | Compound (IIIa-3) | C00321 | 100.6 | 96.4 |
| 8 | Compound (IIIb-2) | C00321 | 100.4 | 94.0 |

As clearly shown in Table 3, compared to the reagents comprising a amphoteric bile acid derivative (to be more specific, cholic acid) and an amphoteric bile acid derivative (to be more specific, CHAPS) as a bile acid derivative, the reagents comprising a nonionic bile acid derivative [to be more specific, BIGCHAP, Compound (IIIa-1), Compound (IIIa-2), Compound (IIIa-3) and Compound (IIIb-2)] as a bile acid derivative showed better stability of the enzyme for the quantitative determination of cholesterol (to be more specific, LPL311 or COO321). It has been known that, generally, stability upon storage of an enzyme and the like at the high temperature of about 40°C reflects the stability upon storage at 4°C, room temperature, etc. Accordingly, it is noted that, when a bile acid derivative having a nonionic surface activity is used as a bile acid derivative in a measuring method and a measuring reagent or measuring kit for quantitatively determining cholesterol in high-density lipoprotein according to the present invention, cholesterol in high-density lipoprotein can be measured more accurately even if a reagent which is stored for a long period is used or even if a reagent which has a risk of being exposed to a storing temperature of 40°C.

### Industrial Applicability

In accordance with the present invention, a simple and accurate method for the quantitative determination of cholesterol in high-density lipoprotein in a sample and a reagent used for the method as well as a reagent and a kit for quantitatively determining cholesterol in high-density lipoprotein where enzyme activity is stably kept even when stored for a long period are provided.

## Claims

1. A method for quantitatively determining cholesterol in high-density lipoprotein, which comprises:
reacting a sample with cholesterol esterase and cholesterol oxidase or cholesterol esterase, an oxidized coenzyme and cholesterol dehydrogenase in an aqueous medium comprising a bile acid derivative; and
measuring the formed hydrogen peroxide or a reduced coenzyme.

2. The method according to claim 1, wherein the aqueous medium further comprises albumin.

3. The method according to claim 1 or 2, wherein the cholesterol esterase is chemically modified cholesterol esterase.

4. The method according to claim 3, wherein the chemically modified cholesterol esterase is cholesterol esterase which is modified by a group selected from the group consisting of a group having poly (ethylene glycol) as a main component, a group having poly(propylene glycol) as a main component, a group having a copolymer of poly(propylene glycol) and poly(ethylene glycol), a group having a water-soluble polysaccharide, a sulfopropyl group, asulfobutylgroup, a polyurethane group and a group having a chelating function.

5. The method according to claim 3, wherein the chemically modified cholesterol esterase is cholesterol esterase which is modified by a group having poly(ethylene glycol) as a main component.

6. The method according to any one of claims 1 to 5, wherein the bile acid derivative is a bile acid derivative having an anionic surface activity.

7. The method according to claim 6, wherein the bile acid derivative having an anionic surface activity is selected from the group consisting of cholic acid or a salt thereof , taurocholic acid or a salt thereof, glycocholic acid or a salt thereof, lithocholic acid or a salt thereof, deoxycholic acid or a salt thereof, chenodeoxycholic acid or a salt thereof, ursodeoxycholic acid or a salt thereof, 7-oxolithocholic acid or a salt thereof, 12-oxolithocholic acid or a salt thereof, 12-oxochenodeoxycholic acid or a salt thereof, 7-oxodeoxycholic acid or a salt thereof, hyocholic acid or a salt thereof, hyodeoxycholic acid or a salt thereof and dehydrocholic acid or a salt thereof.

8. The method according to any one of claims 1 to 5, wherein the bile acid derivative is a bile acid derivative having a amphoteric surface activity.

9. The method according to claim 8, wherein the bile acid derivative having an amphoteric surface activity is a compound represented by the formula (I)
R¹-CH₂-CH(R²)-CH₂-SO₃⁻ (I)
[wherein R¹ is a 3-(3-cholamidopropyl)dimethylammonio group and R² is a hydrogen atom or a hydroxyl group].

10. The method according to any one of claims 1 to 5, wherein the bile acid derivative is a bile acid derivative having a nonionic surface activity.

11. The method according to claim 10, wherein the bile acid derivative having a nonionic surface activity is a compound represented by the formula (II) 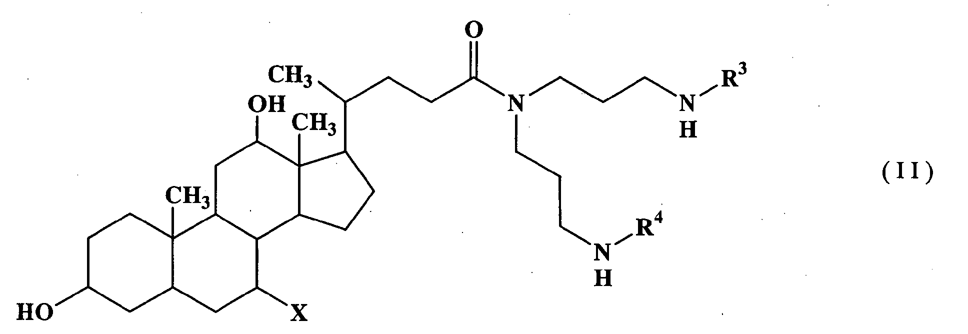 (wherein X is a hydrogen atom or a hydroxyl group; R³ and R⁴ may be the same or different and each represents a substituted or unsubstituted alkyl group or a substituted or unsubstituted alkanoyl group) or a compound represented by the formula (III) 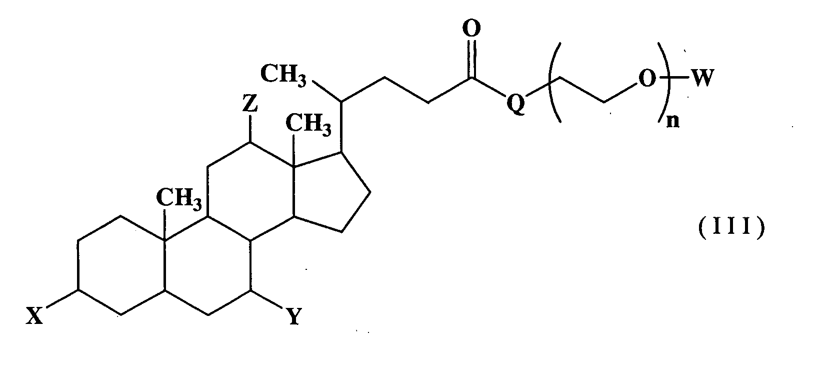 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 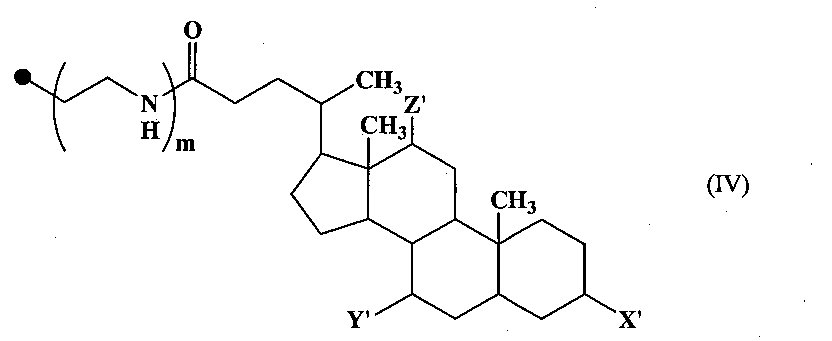 [wherein X', Y' and Z' may be the same or different and each represents a hydrogen atom, a hydroxyl group or an oxo (=O) group; and m is 0 or 1]; and n is an integer of 3 to 400}.

12. A reagent for quantitatively determining cholesterol in high-density lipoprotein, which comprises cholesterol esterase, cholesterol oxidase, a bile acid derivative and a reagent for quantitatively determining hydrogen peroxide.

13. A reagent for quantitatively determining cholesterol in high-density lipoprotein, which comprises cholesterol esterase, cholesterol dehydrogenase, a bile acid derivative and an oxidized coenzyme.

14. The reagent according to claim 13, which further comprises a reagent for quantitatively determining a reduced coenzyme.

15. The reagent according to any one of claims 12 to 14, which further comprises albumin.

16. The reagent according to any one of claims 12 to 15, wherein the cholesterol esterase is chemically modified cholesterol esterase.

17. The reagent according to claim 16, wherein the chemically modified cholesterol esterase is cholesterol esterase which is modified by a group selected from the group consisting of a group having poly(ethylene glycol) as a main component, a group having poly(propylene glycol) as a main component, a group having a copolymer of poly(propylene glycol) and poly(ethylene glycol), a group having a water-soluble polysaccharide, a sulfopropyl group, a sulfobutyl group, a polyurethane group and a group having a chelating function.

18. The reagent according to claim 16, wherein the chemically modified cholesterol esterase is cholesterol esterase which is modified by a group having poly(ethylene glycol) as a main component.

19. The reagent according to any one of claims 12 to 18, wherein the bile acid derivative is a bile acid derivative having an anionic surface activity.

20. The reagent according to claim 19, wherein the bile acid derivative having an anionic surface activity is selected from the group consisting of cholic acid or a salt thereof, taurocholic acid or a salt thereof, glycocholic acid or a salt thereof, lithocholic acid or a salt thereof, deoxycholic acid or a salt thereof, chenodeoxycholic acid or a salt thereof, ursodeoxycholic acid or a salt thereof, 7-oxolithocholic acid or a salt thereof, 12-oxolithocholic acid or a salt thereof, 12-oxochenodeoxycholic acid or a salt thereof, 7-oxodeoxycholic acid or a salt thereof, hyocholic acid or a salt thereof, hyodeoxycholic acid or a salt thereof and dehydrocholic acid or a salt thereof.

21. The reagent according to any one of claims 12 to 18, wherein the bile acid derivative is a bile acid derivative having an amphoteric surface activity.

22. The reagent according to claim 21, wherein the bile acid derivative having an amphoteric surface activity is a compound represented by the formula (I)
R¹-CH₂-CH(R²)-CH₂-SO₃⁻ (I)
[wherein R¹ is a 3-(3-cholamidopropyl)dimethylammonio group and R² is a hydrogen atom or a hydroxyl group].

23. The reagent according to any one of claims 12 to 18, wherein the bile acid derivative is a bile acid derivative having a nonionic surface activity.

24. The reagent according to claim 23, wherein the bile acid derivative having a nonionic surface activity is a compound represented by the formula (II) 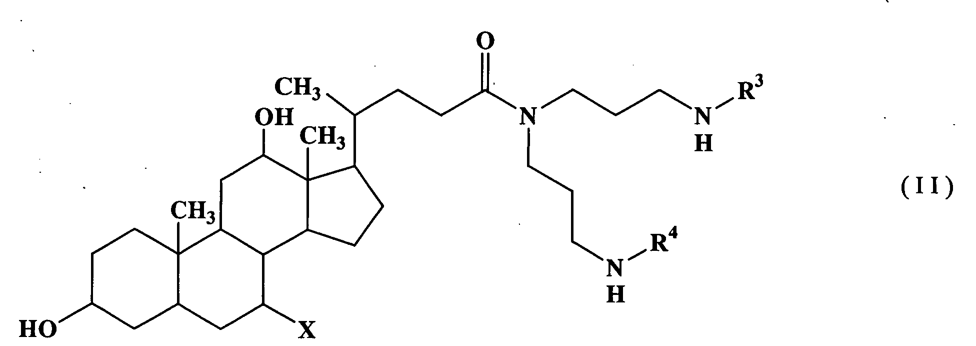 (wherein X is a hydrogen atom or a hydroxyl group; R³ and R⁴ may be the same or different and each represents a substituted or unsubstituted alkyl group or a substituted or unsubstituted alkanoyl group) or a compound represented by the formula (III) 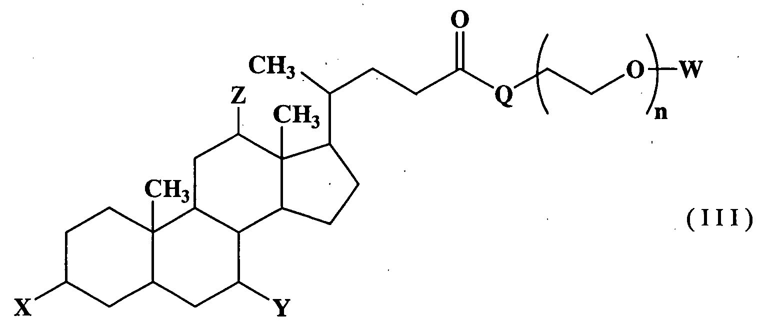 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 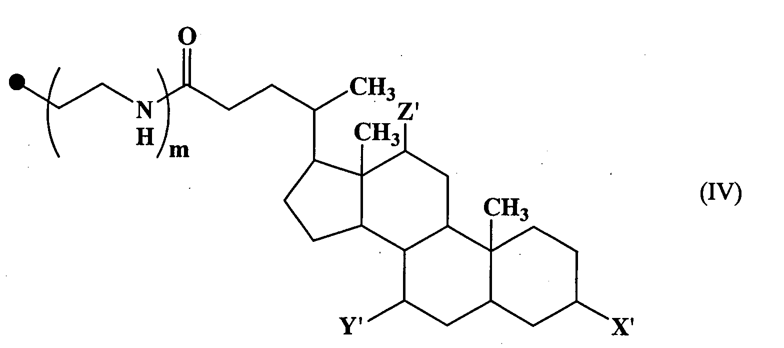 [wherein X', Y' and Z' may be the same or different and each represents a hydrogen atom, a hydroxyl group or an oxo (=O) group; and m is 0 or 1]; and n is an integer of 3 to 400}.

25. A kit for quantitatively determining cholesterol in high-density lipoprotein, which comprises a first reagent comprising cholesterol esterase and a second reagent comprising cholesterol oxidase, wherein a bile acid derivative and a reagent for quantitatively determining hydrogen peroxide are comprised in either or both of the first reagent and/or the second reagent.

26. A kit for quantitatively determining cholesterol in high-density lipoprotein, which comprises a first reagent comprising a bile acid derivative and a second reagent comprising cholesterol esterase and cholesterol oxidase, wherein a reagent for quantitatively determining hydrogen peroxide is comprised in either or both of the first reagent and/or the second reagent.

27. A kit for quantitatively determining cholesterol in high-density lipoprotein, which comprises a first reagent comprising a reagent for quantitatively determining hydrogen peroxide a second reagent comprising cholesterol esterase and cholesterol oxidase wherein a bile acid derivative is comprised in either or both of the first reagent and/or the second reagent.

28. A kit for quantitatively determining cholesterol in high-density lipoprotein, which comprises a first reagent cholesterol esterase and a second reagent comprising cholesterol dehydrogenase where a bile acid derivative and an oxidized coenzyme are comprised in either or both of the first reagent and/or the second reagent.

29. A kit for quantitatively determining cholesterol in high-density lipoprotein, which comprises a first reagent comprising a bile acid derivative and a second reagent comprising cholesterol esterase and cholesterol dehydrogenase wherein an oxidized coenzyme is comprised in either or both of the first reagent and/or the second reagent.

30. The kit according to claim 28 or 29, which further comprises a reagent for quantitatively determining a reduced coenzyme in either or both of the first reagent and/or the second reagent.

31. The kit according to any one of claims 25 to 30, which further comprises albumin in either or both of the first reagent and/or the second reagent.

32. The kit according to any one of claims 25 to 31, wherein the cholesterol esterase is chemically modified cholesterol esterase.

33. The kit according to claim 32, wherein the chemically modified cholesterol esterase is cholesterol esterase which is modified by a group selected from the group consisting of a group having poly (ethylene glycol) as a main component, a group having poly(propylene glycol) as a main component, a group having a copolymer of poly(propylene glycol) and poly(ethylene glycol), a group having a water-soluble polysaccharide, a sulfopropyl group, a sulfobutyl group, a polyurethane group and a group having a chelating function.

34. The kit according to claim 32, wherein the chemically modified cholesterol esterase is cholesterol esterase which is modified by a group having poly(ethylene glycol) as a main component.

35. The kit according to any one of claims 25 to 34, wherein the bile acid derivative is a bile acid derivative having an anionic surface activity.

36. The kit according to claim 35, wherein the bile acid derivative having a anionic surface activity is selected from the group consisting of cholic acid or a salt thereof, taurocholic acid or a salt thereof, glycocholic acid or a salt thereof, lithocholic acid or a salt thereof, deoxycholic acid or a salt thereof, chenodeoxycholic acid or a salt thereof, ursodeoxycholic acid or a salt thereof, 7-oxolithocholic acid or a salt thereof, 12-oxolithocholic acid or a salt thereof, 12-oxochenodeoxycholic acid or a salt thereof, 7-oxodeoxycholic acid or a salt thereof, hyocholic acid or a salt thereof, hyodeoxycholic acid or a salt thereof and dehydrocholic acid or a salt thereof.

37. The kit according to any one of claims 25 to 34 , wherein the bile acid derivative is a bile acid derivative having an amphoteric surface activity.

38. The kit according to claim 37, wherein the bile acid derivative having an amphoteric surface activity is a compound represented by the formula (I)
R¹-CH₂-CH(R²)-CH₂-SO₃⁻ (I)
[wherein R¹ is a 3-(3-cholamidopropyl)dimethylammonio group and R² is a hydrogen atom or a hydroxyl group].

39. The kit according to any one of claims 25 to 34, wherein the bile acid derivative is a bile acid derivative having a nonionic a surface activity.

40. The kit according to claim 39, wherein the bile acid derivative having a nonionic surface activity is a compound represented by the formula (II) 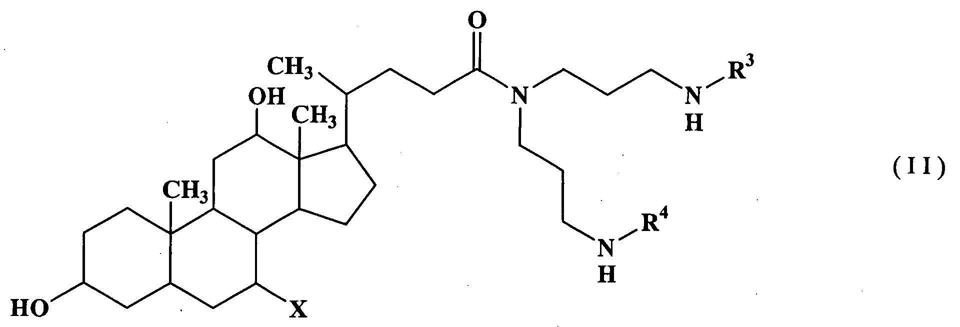 (wherein X is a hydrogen atom or a hydroxyl group; R³ and R⁴ may be the same or different and each represents a substituted or unsubstituted alkyl group or a substituted or unsubstituted alkanoyl group) or a compound represented by the formula (III) 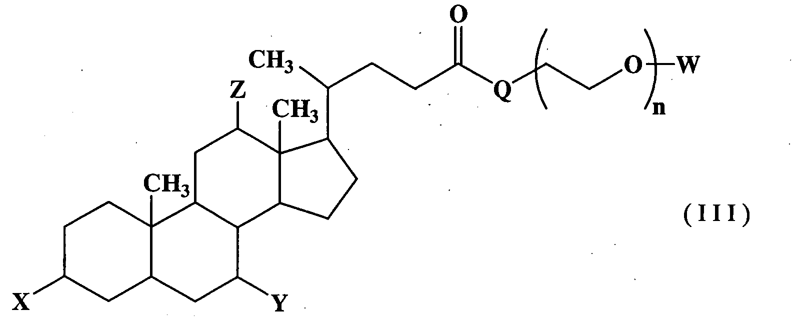 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 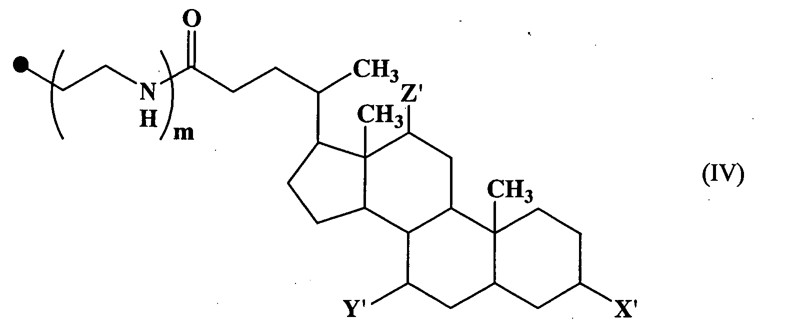 [wherein X', Y' and Z' may be the same or different and each represents a hydrogen atom, a hydroxyl group or an oxo (=O) group; and m is 0 or 1]; and n is an integer of 3 to 400}.

41. A compound represented by the formula (III) 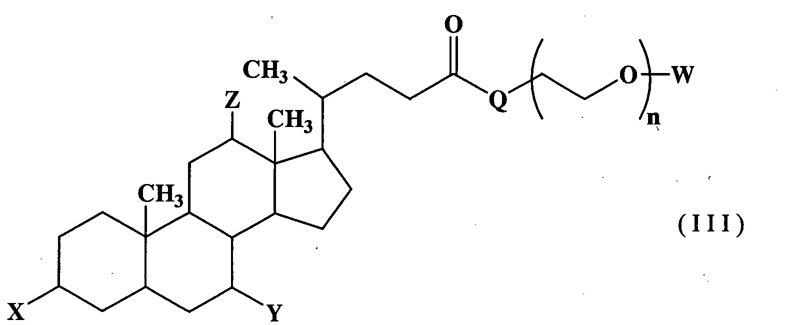 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 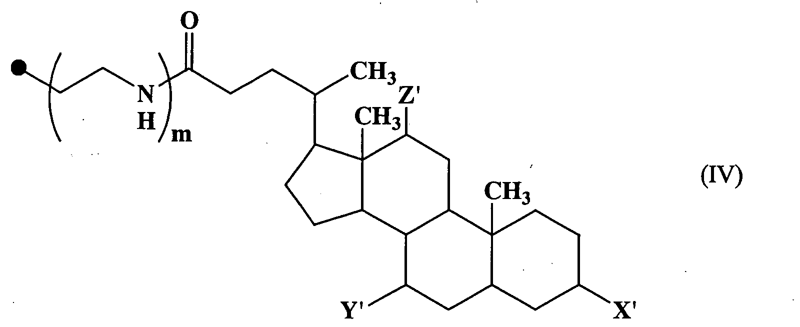 [wherein X', Y' and Z' may be the same or different and each represents a hydrogen atom, a hydroxyl group or an oxo (=O) group; and m is 0 or 1]; and n is an integer of 3 to 400}.

42. A process for producing a compound represented by the formula (III) 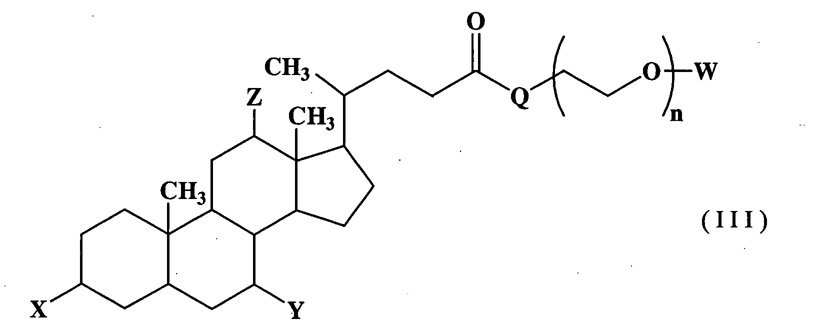 {wherein X, Y and Z may be the same or different and each represents a hydrogen atom, a hydroxyl group or an oxo (=O) group; Q is an oxygen atom or NH; W is a hydrogen atom, an alkyl group, an alkenyl group, an alkynyl group, a cycloalkyl group, a cycloalkenyl group, an alkanoyl group, an alkenoyl group, an alkynoyl group, a substituted or unsubstituted aryl group, a substituted or unsubstituted aminoalkyl group or a group represented by the formula (IV) 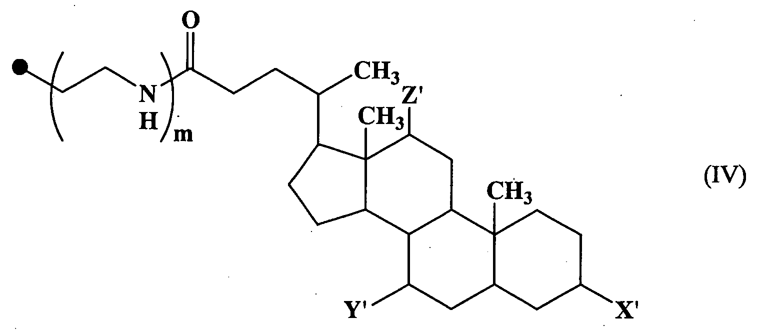 [wherein X', Y' and Z' may be the same or different and each represents a hydrogen atom, a hydroxyl group or an oxo (=O) group; and m is 0 or 1]; and n is an integer of 3 to 400}, which comprises:
reacting a compound represented by the formula (V) 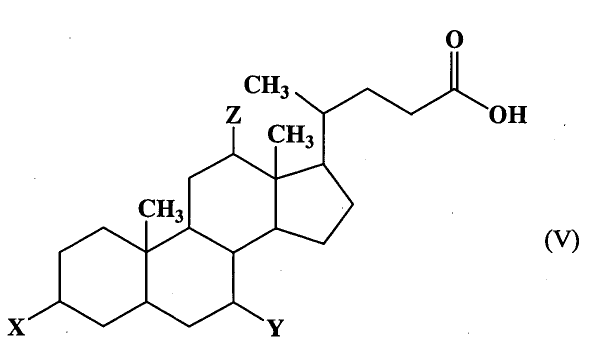
[wherein X, Y and Z may be the same or different and each represents a hydrogen atom, a hydroxyl group or an oxo (=O) group] with a compound represented by the formula (VI) 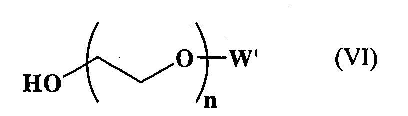 (wherein W' is a hydrogen atom, an alkyl group, an alkenyl group, an alkynyl group, a cycloalkyl group, a cycloalkenyl group, an alkanoyl group, an alkenoyl group, an alkynoyl group or a substituted or unsubstituted aryl group; and n is an integer of 3 to 400) or with a compound represented by the formula (VII) 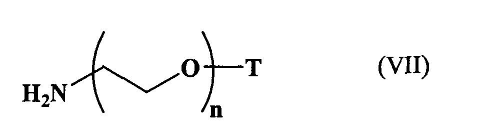 (wherein T is a substituted or unsubstituted aminoalkyl group; and n is an integer of 3 to 400) in the presence of a condensing agent.
